(19) Europäisches Patentamt
European Patent Office
Office européen des brevets

(11) EP 0 445 844 B1

(12) **EUROPEAN PATENT SPECIFICATION**

(45) Date of publication and mention of the grant of the patent:
**06.11.1996 Bulletin 1996/45**

(51) Int Cl.[6]: **C10L 1/24**, C10L 1/14

(21) Application number: **91106084.6**

(22) Date of filing: **23.09.1987**

(54) **Distillate fuel**

Destillat-Kraftstoffe

Distillat combustible

(84) Designated Contracting States:
**AT BE CH DE ES FR GB GR IT LI LU NL SE**

(30) Priority: **24.09.1986 GB 8622959**
**17.08.1987 GB 8719423**

(43) Date of publication of application:
**11.09.1991 Bulletin 1991/37**

(62) Application number of earlier application in accordance with Art. 76 EPC: **87308435.4**

(73) Proprietor: **EXXON CHEMICAL PATENTS INC.**
**Linden, New Jersey 07036-0710 (US)**

(72) Inventors:
- **Lewtas, Kenneth**
  **Wantage, OX12 9EX (GB)**
- **Lehmann, Edwin William**
  **Faringdon, Oxfordshire SN7 8HX (GB)**
- **Tack, Robert Dryden**
  **Marston, Oxford OX3 0HB (GB)**
- **Rossi, Albert**
  **Warren, New Jersey 07060 (US)**

(74) Representative: **Hart, Richard Joseph et al**
**Exxon Chemical Limited,**
**Exxon Chemical Technology Centre,**
**P.O. Box 1,**
**Milton Hill**
**Abingdon, Oxfordshire OX13 6BB (GB)**

(56) References cited:
**EP-A- 0 282 342**
**FR-A- 2 095 712**
**FR-A- 2 426 730**
**US-A- 2 683 736**
**US-A- 2 860 040**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 445 844 B1

## Description

This invention relates to new chemical compounds which are useful as crystal modifiers in fuels especially distillate fuels and the use of these chemicals as distillate fuel additives and to fuels containing the additives.

Long n-alkyl derivatives of difunctional compounds have previously been described as has their use as wax crystal modifiers, to wit alkenyl succinic acid (U.S. 3444082), maleic acid (U.S. 4211534) and phthalic acid (GB 2923645 U. S. 4375973 and U.S. 4402708). Amine salts of certain alkylated aromatic sulphonic acids are described in United Kingdom Patent Specification 1209676 as is their use as antirust additives for turbine oils and hydraulic oils.

We have now found that certain novel compounds are useful as wax crystal modifiers in distillate fuels making possible a significant reduction in the size of the wax crystals formed to below 4000 nanometres sometimes below 2000 nanometres preferably below 1000 nanometres when the modifiers are used alone or in combination with other known wax crystal modifiers.

The present invention therefore provides a distillate fuel boiling in the range of 120° to 500°C containing 0.001 to 0.5 wt.% of a compound of the general formula

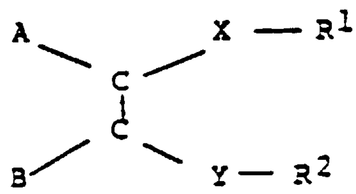

in which

-Y-$R^2$ is $SO_3^{(-)(+)}NR^3_3R^2$, $-SO_3^{(-)(+)}HNR^3_2R^2$, $-SO_3^{(-)(+)}H_2NR^3R^2$, $-SO_3^{(-)(+)}H_3NR^2$, $-SO_2NR^3R^2$ or $-SO_3R^2$;
-X-$R^1$ is -Y-$R^2$ or $-CONR^3R^1$, $-CO_2^{(-)(+)}NR^3_3R^1$, $-CO_2^{(-)(+)}HNR^3_2R^1$, $-CO_2^{(-)(+)}H_2NR^3R^1$, $-CO_2^{(-)(+)}H_3NR^1$, $-R^4-COOR_1$, $-NR^3COR^1$, $R^4OR^1$, $-R^4OCOR^1$, $-R^4R^1$, $-N(COR^3)R^1$ or $Z^{(-)(+)}NR^3_3R^1$;
-$Z^{(-)}$ is $SO_3^{(-)}$ or $-CO_2^{(-)}$;

$R^1$ and $R^2$ are alkyl, alkoxy alkyl or polyalkoxy alkyl containing at least 10 carbon atoms in the main chain;
$R^3$ is hydrocarbyl and each $R^3$ may be the same or different and $R^4$ is nothing or is $C_1$ to $C_5$ alkylene and in

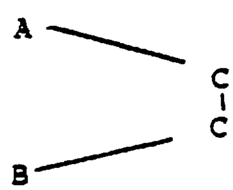

the carbon-carbon (C-C) bond is either a) ethylenically unsaturated when A and B may be alkyl, alkenyl or substituted hydrocarbyl groups or b) part of a cyclic structure which may be aromatic, polynuclear aromatic or cyclo-aliphatic with the proviso that if X-$R^1$ and/or Y-$R^2$ are $-SO_3(-)(+)H_3NR^2$ the distillate fuel does not contain a copolymer of (1) an alpha-olefin having two to seventeen carbon atoms per molecule or an aromatic substituted olefin having eight to forty carbon atoms per molecule and (2) an ester, said ester being a mono- or di-alkyl fumarate, itaconate, citraconate, mesaconate, trans- or cis-glutaconate, in which the alkyl group has 8 to 23 carbon atoms.

It is preferred that -X-$R^1$ and -Y-$R^2$ contain at least three alkyl and/or alkoxy groups.

The ring atoms in such cyclic compounds are preferably carbon atoms, but could, however, include a ring N, S or O atom to give a heterocyclic compound.

Examples of aromatic based compounds from which the additives may be prepared are

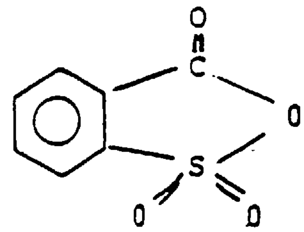

in which the aromatic group may be substituted.

Alternatively they may be obtained from polycyclic compounds, that is those having two or more ring structures which can take various forms. They can be (a) condensed benzene structures, (b) condensed ring structures where none or not all rings are benzene, (c) rings joined "end-on", (d) heterocyclic compounds (e) non-aromatic or partially saturated ring systems or (f) three-dimensional structures.

Condensed benzene structures from which the compounds may be derived include for example naphthalene, anthracene, phenanthrene and pyrene. The condensed ring structures where none or not all rings are benzene include for example Azulene, Indene, Hydroindene, Fluorene, Diphenylene. Compounds where rings are joined end-on include diphenyl.

Suitable heterocyclic compounds from which they may be derived include Quinoline; Indole, 2:3 dihydroindole, benzofuran, coumarin and isocoumarin, benzothiophen, carbazole and thiodiphenylamine.

Suitable non-aromatic or partially saturated ring systems include decalin (decahydronaphthalene), $\alpha$ - pinene, cadinene, bornylene. Suitable 3-dimensional compounds include norbornene, bicycloheptane (norbornane), bicyclo octane and bicyclo octene.

The two substituents must be attached to adjoining ring atoms in the ring when there is only one ring or to adjoining ring atoms in one of the rings where the compound is polycyclic. In the latter case this means that if one were to use naphthalene, these substituents could not be attached to the 1,8- or 4,5- positions, but would have to be attached to the 1,2-, 2,3-, 3,4-, 5,6-, 6,7- or 7,8- positions.

The compounds of the present invention are prepared by reacting both the functional groups in these compounds with amines, alcohols, quaternary ammonium salts etc. Where the compounds are the amides or amine salts they are preferably of a secondary amine which has a hydrogen- and carbon- containing group containing at least 10 carbon atoms. Such amides or salts may be prepared by reacting the acid or anhydride with a secondary amine or alternatively by reacting an amine derivative with a carboxylic acid or anhydride thereof. Removal of water and heating are generally necessary to prepare the amides from the acids. Alternatively the carboxylic acid may be reacted with an alcohol containing at least 10 carbon atoms or a mixture of an alcohol and an amine.

When the compounds are used as fuel additives we prefer that $R^1$ and $R^2$ contain 10 to 22 carbon atoms, for example 14 to 20 carbon atoms and are preferably straight chain or branched at the 1 or 2 position. The other hydrogen- and carbon-containing groups can be shorter e.g. less than 6 carbon atoms or may if desired have at least 10 carbon atoms. Suitable alkyl groups include methyl, ethyl, propyl, hexyl, decyl, dodecyl, tetradecyl eicosyl and docosyl (behenyl).

The especially preferred compounds are the amides or amine salts of secondary amines. Although two substituents are necessary for the cyclic derivatives described above it should be realised that these cyclic compounds can contain one or more further substituents attached to ring atoms of the cyclic compounds.

In a preferred embodiment the groups $R^1$, $R^2$ and $R^3$ are straight chain alkyl groups containing at least 10 carbon atoms.

It is more preferred that the additive is the N, N-dialkyl ammonium salt of 2-(dialkyl-amido)-benzene sulphonic acid or 2-(alkylcarboxy)-benzene sulphonic acid.

These compounds are especially useful as fuel additives especially for mineral oils containing paraffin wax which have the characteristic of becoming less fluid as the temperature of the oil decreases. This loss of fluidity is due to the crystallisation of the wax into plate-like crystals which eventually form a spongy mass entrapping the oil therein. The temperature at which the wax crystals begin to form is known as the Cloud Point and the temperature at which the wax prevents the oil from pouring being the Pour Point.

It has long been known that various additives act as wax crystal modifiers when blended with waxy mineral oils. These compositions modify the size and shape of wax crystals and reduce the cohesive forces between the crystals and between the wax and the oil in such a manner as to permit the oil to remain fluid at lower temperature.

Various Pour Point depressants have been described in the literature and several of these are in commercial use. For example, U.S. Patent No. 3,048,479 teaches the use of copolymers of ethylene and $C_1$-$C_5$ vinyl esters, e.g. vinyl acetate, as pour depressants for fuels, specifically heating oils, diesel and jet fuels. Hydrocarbon polymeric pour depressants based on ethylene and higher alpha-olefins, e.g. propylene, are also known.

U.S. Patent 3,961,916 teaches the use of a mixture of copolymers, to control the size of the wax crystals and United Kingdom Patent 1,263,152 suggests that the size of the wax crystals may be controlled by using a copolymer having a low degree of side chain branching. Both systems improve the ability of the fuel to pass through filters as determined by the Cold Filter Plugging Point (CFPP) test since instead of plate like crystals formed without the presence of additives the needle shaped wax crystals produced will not block the pores of the filter rather forming a porous cake on the filter allowing passage of the remaining fluid.

Other additives have also been proposed for example, United Kingdom Patent 1,469,016, suggests that the copolymers of di-n-alkyl fumarates and vinyl acetate which have previously been used as pour depressant for lubricating oils may be used as co-additives with ethylene/vinyl acetate copolymers in the treatment of distillate fuels with high final boiling points to improve their low temperature flow properties.

U.S. Patent 3,252,771 relates to the use of polymers of $C_{16}$ to $C_{18}$ alpha-olefins obtained by polymerising olefin mixtures that predominate in normal $C_{16}$ to $C_{18}$ alpha-olefins with aluminium trichloride/alkyl halide catalysts as pour depressants in distillate fuels of the broad boiling, easy-to-treat types available in the United States in the early 1960's.

It has also been proposed to use additives based on olefin/maleic anhydride copolymers. For example, U.S. Patent 2,542,542 uses copolymers of olefins such as octadecene with maleic anhydride esterified with an alcohol such as lauryl alcohol as pour depressants and United Kingdom Patent 1,468,588 uses copolymers of $C_{22}$-$C_{28}$ olefins with maleic anhydride esterified with behenyl alcohol as co-additives for distillate fuels.

Similarly, Japanese Patent Publication 5,654,037 uses olefin/maleic anhydride copolymers which have been reacted with amines as pour point depressants and in Japanese Patent Publication 5,654,038 the derivatives of the olefin/maleic anhydride copolymers are used together with conventional middle distillate flow improvers such as ethylene vinyl acetate copolymers.

Japanese Patent Publication 5,540,640 discloses the use of olefin/maleic anhydride copolymers (not esterified) and states that the olefins used should contain more than 20 carbon atoms to obtain CFPP activity.

United Kingdom 2,192,012 uses mixtures of esterified olefin/maleic anhydride copolymers and low molecular weight polyethylene, the esterified copolymers being ineffective when used as sole additives. The patent specifies that the olefin should contain 10-30 carbon atoms and the alcohol 6-28 carbon atoms with the longest chain in the alcohol containing 22-40 carbon atoms.

United States Patents 3,444,082; 4,211,534; 4,375,973 and 4,402,708 discussed previously suggest the use of certain nitrogen containing compounds.

FR-A-2 426 730 discloses a distillate fuel which contains 0.001 to 0.5 wt.% of an additive composition which in turn comprises 0.1 to 10 wt.% of a polar oil-soluble compound (component C) acting as an anti-agglomerant for wax particles. The polar compound is monomeric and may be ionic or nonionic. When ionic, it comprises as anion an oil-soluble group $R^5Y$ and as cation a mono-, di-, tri-or tetra-alkyl ammonium group, i.e. $R^7ZH_3^+$, $(R^7)_2ZH_2^+$, $(R^7)_3ZH^+$ or $(R^7)_4Z^+$. According to the definitions of $R^5$ and Y of formula $R^5Y$, wherein Y is the polar end group and $R^5$ is the oil-solubilizing group, given on page 12, line 18 to page 13, line 33, Y may be a sulphonate group, sulphate group, phenate group or borate group, and $R^5$ may be aliphatic, cycloaliphatic or aromatic and is preferably alkyl, alkaryl or alkenyl. The alkyl group can have a carbon content of 8 to 150 carbon atoms. In particular the anion may be an alkaryl sulfonate having a long-chain alkyl ammonium cation.

The improvement in CFPP activity achieved by the incorporation of the additives of these Patents is achieved by modifying the size and shape of the wax crystals forming to produce needle like crystals generally of particle size 10000 nanometres or bigger typically 30000 to 100000 nanometres. In operation of diesel engines or heating systems at low temperatures, these crystals do not generally pass through the filters but form a permeable cake on the filter allowing the liquid fuel to pass; the wax crystals will subsequently dissolve as the engine and the fuel heats up, which can be by the bulk fuel being heated by recycled fuel. This can, however, result in the wax crystals blocking the filters, leading to starting problems and problems at the start of driving in cold weather or failure of fuel heating systems.

We have found that by using the compounds of the present invention particularly small wax crystals may be obtained which will pass through the filters of typical diesel engines and heating systems rather than forming a cake or the filter.

We have also found that by using the compounds of the present invention the production of small crystals reduces the tendency of the wax crystals to settle in the fuel during storage and can also result in a further improvement in the CFPP performance of the fuel.

The amount of the compound added to the distillate fuel oil is 0.001 to 0.5 wt.%, for example 0.01 to 0.10 wt.% based on the weight of fuel.

The compound may conveniently be dissolved in a suitable solvent to form a concentrate of from 20 to 90, e.g. 30 to 80 weight % in the solvent. Suitable solvents include kerosene, aromatic naphthas, mineral lubricating oils etc.

The use of the additives of this invention allows distillate fuel oil boiling in the range 120°C to 500°C and which has a wax content of at least 0.5 wt. % at a temperature of 10°C below the wax appearance temperature, to be produced with wax crystals at that temperature having an average particle size less than 4000 nanometres, sometimes less than

2000 nanometres and depending on the fuel, the crystals can be less than 1000 nanometres.

The Wax Appearance Temperature (WAT) of the fuel is measured by differential scanning calorimetry (DSC). In this test a small sample of fuel (25 ul) is cooled at 2°C/minute together with a reference sample of similar thermal capacity but which will not precipitate wax in the temperature range of interest (such as kerosene). An exotherm is observed when crystallisation commences in the sample. For example the WAT of the fuel may be measured by the extrapolation technique on the Mettler TA 2000B.

The wax content is derived from the DSC trace by integrating the area enclosed by the baseline and the exotherm down to the specified temperature. The calibration having been previously performed on a known amount of crystallizing wax.

The wax crystal average particle size is measured by analysing a Scanning Electron Micrograph of a fuel sample at a magnification of 4000 to 8000 X and measuring the longest axis of 50 crystals over a predetermined grid. We find that, provided the average size is less than 4000 nanometres, the wax will begin to pass through the typical paper filters used in diesel engines together with the fuel although we prefer that the size be below 3000 nanometres, more preferably below 2000 and most preferably below 1000 nanometres. The actual size attainable depends upon the original nature of the fuel and the nature and amount of additive used but we have found that these sizes and smaller are attainable.

The ability to obtain such small wax crystals in the fuel shows significant benefit in diesel engine operability as shown by pumping previously stirred (to remove settled wax effects) fuel through a diesel filter at from 8 to 15 ml/second and 1.0 to 2.4 litres per minute per square metre of filter surface area at a temperature at least 5°C below the wax appearance temperature with at least 1 wt.% of the fuel being present in the form of solid wax. Both fuel and wax are considered to successfully pass through the filter if one or more of the following criteria are satisfied:

(i) When 18 to 20 litres of fuel have passed through the filter the pressure drop across the filter does not exceed 50 KPa, preferably 25 KPa, more preferably 10 KPa, most preferably 5 KPa.

(ii) At least 60%, preferably at least 80%, more preferably at least 90 wt.% of the wax present in the fuel, as determined by the DSC test is found to be present in the fuel leaving the filter.

(iii) Whilst pumping 18 to 20 litres of fuel through the filter, the flow rate always remains at above 60% of the initial flow rate and preferably above 80%.

These fuels containing the compounds of this invention have outstanding benefits compared to previous distillate fuels improved in their cold flow properties by the addition of conventional additives. For example the fuels are operable at temperatures approaching the pour point and not restricted by the inability to pass the CFPP test. Hence these fuels either pass the CFPP test at significantly lower temperatures or obviate the need to pass that test. The fuels also have improved cold start performance at low temperatures since they do not rely on recirculation of warm fuel to dissolve undesirable wax deposits. The fuels also have a reduced tendency for the wax crystals to settle in the fuel during storage reducing the tendency for wax to agglomerate at the bottom of storage vessels so blocking filters, etc.

The best effect is usually obtained when the compounds of the invention are used in combination with other additives known for improving the cold flow properties of distillate fuels generally, although they may be used on their own.

The compounds are preferably used together with what are known as comb polymers of the general formula

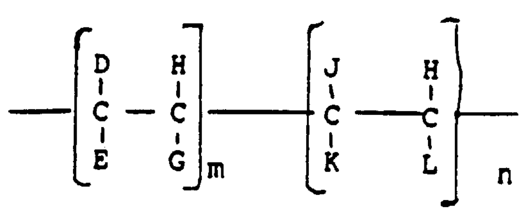

where

D = R, CO.OR, OCO.R, R'CO.OR or OR
E = H or $CH_3$ or D or R'
G = H, or D
m = 1.0 (homopolymer) to 0.4 (mole ratio)
J = H, R', Aryl or Heterocyclic group, R'CO.OR K = H, CO.OR', OCO.R', OR', $CO_2H$

L = H, R', CO.OR', OCO.R', Aryl, $CO_2H$ .
n = 0.0 to 0.6 (mole ratio)
$R \geq C_{10}$
$R' \geq C_1$

Another monomer may be terpolymerized if necessary

Examples of suitable comb polymers are the fumarate/vinyl acetate particularly those described in our European Patent Applications 0153176, 0153177, 85301047 and 85301048 and esterified olefine/maleic anhydride copolymers and the polymers and copolymers of alpha olefines and esterified copolymers of styrene and maleic anhydride.

Examples of other additives with which the compounds of the present invention may be used are the polyoxyalkylene esters, ethers, ester/ethers and mixtures thereof, particularly those containing at least one, preferably at least two $C_{10}$ to $C_{30}$ linear saturated alkyl groups and a polyoxyalkylene glycol group of molecular weight 100 to 5,000 preferably 200 to 5,000, the alkyl group in said polyoxyalkylene glycol containing from 1 to 4 carbon atoms. These materials form the subject of European Patent Publication 0,061,895 A2. Other such additives are described in United States Patent 4,491,455.

The preferred esters, ethers or ester/ethers which may be used may be structurally depicted by the formula:

R-O(A)-O-R"

where R and R" are the same or different and may be

i) n-alkyl

ii) 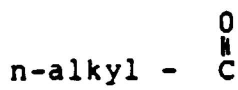

iii) 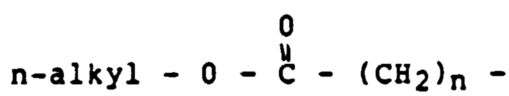

iv) 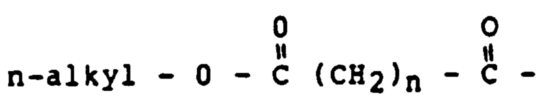

the alkyl group being linear and saturated and containing 10 to 30 carbon atoms, and A represents the polyoxyalkylene segment of the glycol in which the alkylene group has 1 to 4 carbon atoms, such as polyoxymethylene, polyoxyethylene or polyoxytrimethylene moiety which is substantially linear; some degree of branching with lower alkyl side chains (such as in polyoxypropylene glycol) may be tolerated but it is preferred the glycol should be substantially linear, A may also contain nitrogen.

Suitable glycols generally are the substantially linear polyethylene glycols (PEG) and polypropylene glycols (PPG) having a molecular weight of about 100 to 5,000, preferably about 200 to 2,000. Esters are preferred and fatty acids containing from 10-30 carbon atoms are useful for reacting with the glycols to form the ester additives and it is preferred to use a $C_{18}$-$C_{24}$ fatty acid, especially behenic acids. The esters may also be prepared by esterifying polyethoxylated fatty acids or polyethoxylated alcohols.

Polyoxyalkylene diesters, diethers, ether/esters and mixtures thereof are suitable as additives with diesters preferred for use in narrow boiling distillates whilst minor amounts of monoethers and monoesters may also be present and are often formed in the manufacturing process. It is important for additive performance that a major amount of the dialkyl compound is present. In particular, stearic or behenic diesters of polyethylene glycol, polypropylene glycol or polyethylene/polypropylene glycol mixtures are preferred.

The compounds of this invention may also be used with ethylene unsaturated ester copolymer flow improvers. The unsaturated monomers which may be copolymerised with ethylene include unsaturated mono and diesters of the general formula:

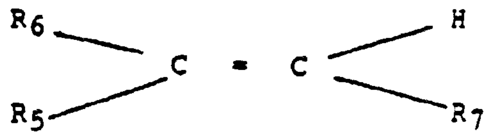

wherein $R_6$ is hydrogen or methyl, $R_5$ is a $-OOCR_8$ group wherein $R_8$ is hydrogen or a $C_1$ to $C_{28}$, more usually $C_1$ to $C_{17}$, and preferably a $C_1$ to $C_8$, straight or branched chain alkyl group; or $R_5$ is a $-COOR_8$ group wherein $R_8$ is as previously described but is not hydrogen and $R_7$ is hydrogen or $-COOR_8$ as previously defined. The monomer, when R6 and $R_7$ are hydrogen and R5 is $-OOCR_8$, includes vinyl alcohol esters of $C_1$ to $C_{29}$, more usually $C_1$ to C5, monocarboxylic acid, and preferably $C_2$ to $C_{29}$, more usually $C_1{}^2$ to C5 monocarboxylic acid, and preferably $C_2$ to $C_5$ monocarboxylic acid. Examples of vinyl esters which may be copolymerised with ethylene include vinyl acetate, vinyl propionate and vinyl butyrate or isobutyrate, vinyl acetate being preferred. We prefer that the copolymers contain from 5 to 40 wt.% of the vinyl ester, more preferably from 10 to 35 wt.% vinyl ester. They may also be mixtures of two copolymers such as those described in US Patent 3,961,916. It is preferred that these copolymers have a number average molecular weight as measured by vapour phase osmometry of 1,000 to 10,000, preferably 1,000 to 5,000.

The compounds of the invention may also be used in distillate fuels in combination with other polar compounds, either ionic or non-ionic, which have the capability in fuels of acting as wax crystal growth inhibitors. Polar nitrogen containing compounds have been found to be especially effective when used in combination with the glycol esters, ethers or ester/ethers and such three component mixtures are within the scope of the present invention. These polar compounds are generally amine salts and/or amides formed by reaction of at least one molar proportion of hydrocarbyl substituted amines with a molar proportion of hydrocarbyl acid having 1 to 4 carboxylic acid groups or their anhydrides; ester/amides may also be used containing 30 to 300, preferably 50 to 150 total carbon atoms. These nitrogen compounds are described in US Patent 4,211,534. Suitable amines are usually long chain $C_{12}$-$C_{40}$ primary, secondary, tertiary or quaternary amines or mixtures thereof but shorter chain amines may be used provided the resulting nitrogen compound is oil soluble and therefore normally containing about 30 to 300 total carbon atoms. The nitrogen compound preferably contains at least one straight chain $C_8$ to C40, preferably $C_{14}$ to $C_{24}$ alkyl segment.

Suitable amines include primary, secondary, tertiary or quaternary, but preferably are secondary. Tertiary and quaternary amines can only form amine salts. Examples of amines include tetradecyl amine, cocoamine, hydrogenated tallow amine and the like. Examples of secondary amines include dioctacedyl amine, methyl-behenyl amine and the like. Amine mixtures are also suitable and many amines derived from natural materials are mixtures. The preferred amine is a secondary hydrogenated tallow amine of the formula $HNR_1R_2$ where in $R_1$ and $R_2$ are alkyl groups derived from hydrogenated tallow fat composed of approximately 4% $C_{14}$, 31% $C_{16}$, 59% $C_{18}$.

Examples of suitable carboxylic acids and their anhydrides for preparing these nitrogen compounds include cyclohexane, 1,2 dicarboxylic acid, cyclohexene, 1,2-dicarboxylic acid, cyclopentane 1,2 dicarboxylic acid, naphthalene dicarboxylic acid and the like. Generally, these acids will have about 5-13 cabon atoms in the cyclic moiety. Preferred acids useful in the present invention are benzene dicarboxylic acids such as phthalic acid, isophthalic acid, and terephthalic acid. Phthalic acid or its anhydride is particularly preferred. The particularly preferred compound is the amide-amine salt formed by reacting 1 molar portion of phthalic anhydride with 2 molar portions of di-hydrogenated tallow amine. Another preferred compound is the diamide formed by dehydrating this amide-amine salt.

Hydrocarbon polymers may also be used as part of the additive combination which may be represented with the following general formula:

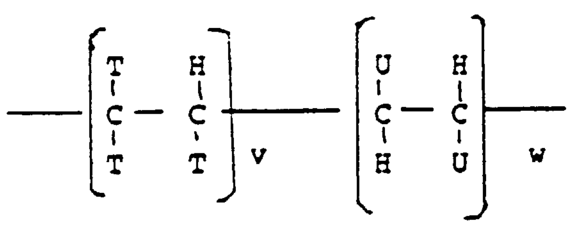

where

T = H or R'
U = H, T or Aryl
v = 1.0 to 0.0 (mole ratio)

w = 0.0 to 1.0 (mole ratio)

where R' is alkyl.

These polymers may be made directly from ethylenically unsaturated monomers or indirectly by hydrogenating the polymer made from monomers such as isoprene, butadiene etc. Preferably one of the other fuel additives is an olefine copolymer. Preferred one ethylene and a $C_3$ to $C_6$ Olefin. A particularly preferred hydrocarbon polymer is a copolymer of ethylene and propylene having an ethylene content preferably between 20 and 60% (w/w) and is commonly made via homogeneous catalysis.

The ratios of additives to be used will depend on the fuel to be treated but generally from 30 to 60 wt.% of the additives is the compound of the invention.

The additive systems which form part of the present invention may conveniently be supplied as concentrates for incorporation into the bulk distillate fuel. These concentrates may also contain other additives as required. These concentrates preferably contain from 3 to 75 wt.%, more preferably 3 to 60 wt.%, most preferably 10 to 50 wt.% of the additives, preferably in solution in oil. Such concentrates are also within the scope of the present invention. The additives of this invention may be used in the broad range of distillate fuels boiling in the range 120° to 500°C.

The invention is illustrated by the following Examples.

Preparation

Example 1

The N,N-dialkyl ammonium salt of 2-dialkylamido benzene sulphonate where the alkyl groups are $nC_{16-18}H_{33-37}$ was prepared by reacting 1 mole of ortho-sulphobenzoic acid cyclic anhydride with 2 moles of di-(hydrogenated) tallow amine in a xylene solvent at 50% (w/w) concentration. The reaction mixture was stirred at between 100°C and the refluxing temperature. The solvent and chemicals should be kept as dry as possible so as not to enable hydrolysis of the anhydride.

The product was analysed by 500 MHz Nuclear Magnetic Resonance Spectroscopy and the spectrum which is attached hereto as Figure 1 confirmed the structure to be

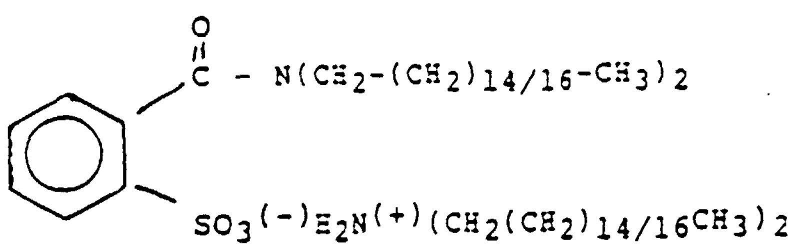

Example 2

Example 1 was repeated except that the ortho-sulphobenzoic acid was reacted first with 1 mole of octadecan-1-ol and 1 mole of di-hydrogenated tallow amine.The product was analysed by 500 MHz N.M.R. and the spectrum is attached hereto as Figure 2 showing the structure to be

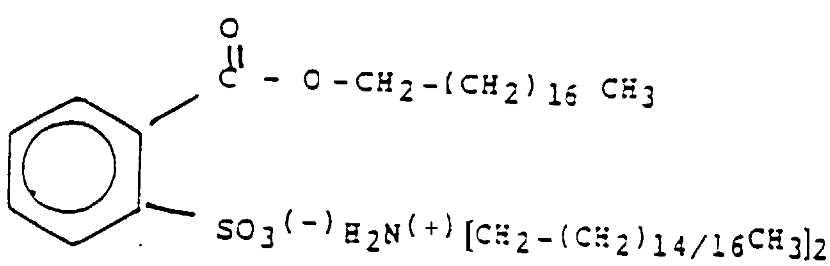

Testing

The effectiveness of the product of Example 1 and additive systems containing the product as filterability improvers

in distillate fuels were determined by the following methods.

By one method, the response of the oil to the additives was measured by the Cold Filter Plugging Point Test (CFPP) which is carried out by the procedure described in detail in "Journal of the Institute of Petroleum", Volume 52, Number 510, June 1966, pp. 173-285. This test is designed to correlate with the cold flow of a middle distillate in automotive diesels.

In brief, a 40 ml. sample of the oil to be tested is cooled in a bath which is maintained at about -34°C to give non-linear cooling at about 1°C/min. Periodically (at each one degree C starting from above the cloud point), the cooled oil is tested for its ability to flow through a fine screen in a prescribed time period using a test device which is a pipette to whose lower end is attached an inverted funnel which is positioned below the surface of the oil to be tested. Stretched across the mouth of the funnel is a 0.045 mm sieve opening (350 mesh) screen having an area defined by a 12 millimetre diameter. The periodic tests are each initiated by applying a vacuum to the upper end of the pipette whereby oil is drawn through the screen up into the pipette to a mark indicating 20 ml. of oil. After each successful passage, the oil is returned immediately to the CFPP tube. The test is repeated with each one degree drop in temperature until the oil fails to fill the pipette within 60 seconds. This temperature is reported as the CFPP temperature. The difference between the CFPP of an additive free fuel and of the same fuel containing additive is reported as the CFPP depression by the additive. A more effective flow improver gives a greater CFPP depression at the same concentration of additive.

Another determination of flow improver effectiveness is made under conditions of the flow improver Programmed Cooling Test (PCT) which is a slow cooling test designed to idicate whether the wax in the fuel will pass through filters such as are found in heating oil distribution system.

In the test, the cold flow properties of the described fuels containing the additives were determined as follows. 300 ml. of fuel are cooled linearly at 1°C/hour to the test temperature and the temperature then held constant. After 2 hours at -9°C, approximately 20 ml. of the surface layer is removed as the abnormally large wax crystals which tend to form on the oil/airinterface during cooling. Wax which has settled in the bottle is dispersed by gentle stirring, then a CFPP filter assembly is inserted. The tap is opened to apply a vacuum of 500 mm. of mercury and closed when 200 ml. of fuel have passed through the filter into the graduated receiver. A PASS is recorded if the 200 ml. are collected within ten seconds through a given mesh size or a FAIL if the flow rate is too slow indicating that the filter has become blocked.

CFPP filter assemblies with filter screens of 0.841, 0.500, 0.420, 0.250, 0.177, 0.150, 0.125, 0.105, 0.075, 0.063 and 0.045 mm (20, 30, 40 60, 80, 100, 120, 150, 200, 250 and 350 mesh number) are used to determine the finest mesh (largest mesh number) the fuel will pass. The larger the mesh number that a wax containing fuel will pass, the smaller are the wax crystals and the greater the effectiveness of the additive flow improver. It should be noted that no two fuels will give exactly the same test results at the same treatment level for the same flow improver additive.

Wax settling studies were also performed prior to PCT filtration. The extent of the setled layer was visually measured as a % of the total fuel volume. This extensive wax settling would be given by a low number whilst an unsettled fluid fuel would be at a state of 100%. Care must be taken because poor samples of gelled fuel with large wax crystals almost always exhibit high values, therefore these results should be recorded as "gel".

The effectiveness of the additives of the present invention in lowering the Cloud Point of distillate fuels was determined by the standard Cloud Point Test (IP-219 or ASTM-D 2500) other measures of the onset of crystallisation are the Wax Appearance Point (WAP) Test (ASTM D.3117-72) and the Wax Appearance Temperature (WAT) as measured by different scanning calorimetry using a Mettler TA 2000B differential scanning calorimeter. In the test a 25 microlitre sample of the fuel is cooled at 2°C/min. from a temperature at least 30°C above the expected cloud point of the fuel. The observed onset of crystallisation is estimated, without correction for thermal lag (approximately 2°C), as the wax appearance temperature as indicated by the differential scanning calorimeter.

The ability of the fuel to pass through a diesel vehicle main filter was determined in an apparatus consisting of a typical diesel vehicle main filter mounted in a standard housing in a fuel line; the Bosch Type as used in a 1980 VW Golf diesel passanger car, and a Cummins FF105 as used in the Cummins NTC engine series are appropriate. A reservoir and feed system capable of supplying half a normal fuel tank of fuel linked to a fuel injection pump as used in the VW Golf is used to draw fuel through the filter from the tank at constant flowrate, as in the vehicle. Instruments are provided to measure pressure drop across the filter, the flow rate from the injection pump and the unit temperatures. Receptables are provided to receive the pumped fuel, both "injected" fuel and the surplus fuel.

In the test the tank is filled with 19 Kilogrammes of fuel and leak tested. When satisfactory, the temperature is stabilised at an air temperature 8°C above fuel cloud point. The unit is then cooled at 3°C/hour to the desired test temperature, and held for at least 3 hours for fuel temperature to stabilise. The tank is vigorously shaken to fully disperse the wax present; a sample is taken from the tank and 1 litre of fuel removed through a sample point on the discharge line immediately after the tank and returned to the tank. The pump is then started, with pump rpm set to equate to pump rpm at a 110 km/h road speed. In the case of the VW Golf this is 1900 rpm, corresponding to an engine speed of 3800 rpm. Pressure drop across the filter and flow rate of fuel from the injection pump are monitored until fuel is exhausted, typically 30 to 35 minutes..

Providing fuel feed to the injectors can be held at 2 ml/sec (surplus fuel will be about 6.5 - 7 ml/sec) the result is

a "PASS". A drop in feed fuel flow to the injectors signifies a "BORDERLINE" result; zero flow a "FAIL".

Typically, a "PASS" result may be associated with an increasing pressure drop across the filter, which may rise as high as 60 kPA. Generally considerable proportions of wax must pass the filter for such a result to be achieved. A "GOOD PASS" is characterised by a run where the pressure drop across the filter does not rise above 10 kPa, and is the first indication that most of the wax has passed through the filter, an excellent result has a pressure drop below 5kPa.

Additionally, fuel samples are taken from "surplus" fuel and "injector feed" fuel, ideally every four minutes throughout the test. These samples, together with the pre-test tank samples, are compared by DSC to establish the proportion of feed wax that has passed through the filter. Samples of the pre-test fuel are also taken and SEM samples prepared from them after the test to compare wax crystal size and type with actual performance.

The following additives were used

(i) The Product of Example 1

(ii) Additives A

A1 is a 1:3 (w/w) mixture of two ethylene-vinyl acetate copolymers; A3 consisting of ethylene and about 38 wt.% vinyl acetate, and has a number average molecular weight of about 1800 (VPO) and A2 consisting of ethylene and about 17 wt.% vinyl acetate and has a number average molecular weight of about 3000 (VPO). A4 is a 50/50% mixture of A2 and A3.

A5 consists of a polymer containing 13.5 wt.% vinyl acetate and has a number average molecular weight of 3500 (VPO).

(iii) Additive B

Polyethylene glycol (PEG) esters and polypropylene glycol (PPG) esters were prepared by mixing one molar proportion of the polyethylene or polypropylene glycol with one or two molar proportions of the carboxylic acids for the "mono" and "di-" esters respectively. Para-toluene sulphonic acid was added at 0.5 wt.% of the reactant mass as catalyst. The mixture was heated to 150°C with stirring and a slow stream of nitrogen to distil off water of reaction. When the reaction was completed, as judged by the infrared spectrum, the product was poured out while molten and allowed to cool, giving a waxy solid.

PEG's and PPG's are usually referred to in combination with their molecular weights, e.g. PEG 600 is a 600 average molecular weight polyethylene glycol. This nomenclature has been continued here so PEG 600 dibehenate is the ester product of the reaction of two molar proportions of behenic acid with one mole of PEG 600 which is Additive B used herein.

(iv) Additive C

The reaction product of one mole of phthalic anhydride with two moles of dihydrogenated tallow amine, to form a half amide/half amine salt.

(v) Additive D

A copolymer of ethylene and propylene containing 56 wt.% ethylene and of number average molecular weight of 60,000.

(vi) Additives E

$E_1$ was made by esterifying a 1:1 molar styrene-maleic anhydride copolymer with 2 moles of a 1:1 molar mixture of $C_{12}H_{25}OH$ and $C_{14}H_{29}OH$ per mole of anhydride groups were used in the esterification (slight excess, approximately 5% alcohol used) step using p-toluene sulphonic acid as the catalyst (1/10 mole) in xylene solvent. which gave a molecular weight (Mn) of 50,000 and contained 3% (w/w) untreated alochol.

Polymer E2 was created by using 2 moles of the $C_{14}H_{29}OH$ to esterify the styrene maleic anhydride copolymer and this too gave a number average molecular weight of 50,000 and contained 3.3% (w/w) free alcohol.

In these further Examples fuels were treated with the additives, then cooled to 10°C below their Wax Appearance Temperature (WAT), and the wax crystal size measured from an electron scanning micrograph and the ability of the fuel to pass through a Cummins FT105 fuel filter was determined. The results were as follows.

Example 3

Characteristics of Fuel used

| | |
|---|---|
| Cloud Point | -14°C |
| Untreated CFPP | -16°C |
| Wax Appearance Temperature | -18.6°C |
| Initial Boiling Point | 178°C |
| 20% | 230°C |
| 90% | 318°C |
| Final Boiling Point | 355°C |
| Wax content at -25°C | 1.1 wt.% |

An additive combination comprising 250 p.p.m. of each of the Product of Example I, Additives A5 and $E_1$ were included in the fuels and tested at -25°C and the wax crystal size was found to be 1200 nanometres long and above 90 wt.% of the wax passed through the Cummins FF105 filter.

During the test, passage of wax was further evidenced by observing the pressure drop over the filter, which only increased by 2.2 kPa.

Example 4

Example 3 was repeated and the wax crystal size was found to be 1300 nanometres and the maximum pressure drop across the filter was 3.4 Kpa.

Example 5

Characteristics of Fuel Used

| | |
|---|---|
| Cloud Point | 0°C |
| Untreated CFPP | -5°C |
| Wax Appearance Temperature | -2.5°C |
| Initial Boiling Point | 182°C |
| 20% | 220°C |
| 90% | 354°C |
| Final Boiling Point | 385°C |
| Wax content at test temperature | 1.6 wt%. |

An additive combination of 250 p.p.m. of each of the Product of Example 1 and Additives A5 and $E_2$ were incorporated in the fuel and the wax crystal size was found to be 1500 nanometres and about 75 wt% of the wax passed through the Bosch 145434106 filter at the test temperature of -8.5°C. The maximum pressure drop across the filter was 6.5 Kpa.

Example 6

Example 5 was repeated and found to give wax crystal size 2000 nanometres long of which about 50 wt.% passed through filter giving a maximum pressure drop of 35.3 Kpa.

Example 7

The fuel used in Example 5 was treated with 400 ppm of the product of Example 1 and 100 ppm of A1, the fuel was tested as in Example 5 at -8ºC at which temperature the wax content 1.4 wt.%. The wax crystal size was found to be 2500 nanometres and 50 wt.% of the wax passed through the filter with a pressure drop of 67.1 Kpa.

When using this fuel in the test rig, pressure drop rose rather quickly and the test failed. We believe this is because as shown in the photograph the crystals are flat and flat crystals that fail to pass the filter tend to cover the filter with a thin, impermeable layer. "Cube like" (or "nodular") crystals on the other hand when not passing through the filters, collect in a comparatively loose "cake", through which fuel can still pass until the mass becomes so great that the filter fills and the total thickness of wax "cake" is so great that the pressure drop again is excessive.

Example 8 (Comparative)

The fuel used in Example 5 was treated with 500 ppm of a mixture of 4 parts of Additive C and 1 part of Additive A5 and tested at -8°C, the wax crystal size was found to be 6300 nanometres and 13 wt.% of the wax passed through the filter.

This example is among the very best examples of the prior art, but without crystal passage.

A scanning electron micrograph of the wax crystals forming in the fuel of Examples 3 to 8 are Figures 3 to 8 hereof. These were prepared by placing samples of fuel in 60 ml (2 oz.) bottles in cold boxes held about 8°C above fuel cloud point for 1 hour while fuel temperature stabilises. The box is then cooled at 1°C an hour down to the test temperature, which is then held.

A pre-prepared filter carrier, consisting of a 10 mm diameter sintered ring, surrounded with a 1 mm wide annular metal ring, supporting a 200 nanometres rated silver membrane filter which is held in position by two vertical pins, is then placed on a vacuum unit. A vacuum of at least 30 kPa is applied, and the cooled fuel dripped onto the membrane from a clean dropping pipette until a small domed puddle just covers the membrane. The fuel is dropped slowly to sustain the puddle; after about 10-20 drops of fuel have been applied the puddle is allowed to drain down leaving a thin dull matt layer of fuel wet wax cake on the membrane. A thick layer of wax will not wash acceptably, and a thin one may be washed away. The optimal layer thickness is a function of crystal shape, with "leafy" crystals needing thinner layers than "nodular" crystals. It is important that the final cake have a matt appearance. A "shiny" cake indicates excessive residual fuel and crystal "smearing" and should be discarded.

The cake is then washed with a few drops of methyl ethyl ketone which are allowed to completely drain away. The process is repeated a number of times. When washing is complete the methyl ethyl ketone will disappear very quickly, leaving a "brilliant matt white" surface which will turn grey on application of another drop of methyl ethyl ketone.

The washed sample is then placed in a cold dessicator, and kept until ready for coating in the SEM. It may be necessary to keep the sample refrigerated to preserve the wax, in which case it should be stored in a cold box prior to transfer (in a suitable sample transfer container) to the SEM to avoid ice crystal formation on the sample surface.

During coating, the sample must be kept as cold as possible to minimise damage to the crystals. Electrical contact with the stage is best provided for by a retaining screw pressing the annular ring against the side of a well in the stage designed to permit the sample surface to lie on the instrument focal plane. Electrically conductive paint can also be used.

Once coated, the micrographs are obtained in a conventional way on the Scanning Electron Microscope. The photomicrographs are analysed to determine the average crystal size by fastening a transparent sheet with 88 points marked (as dots) at the intersections of a regular, evenly spaced grid 8 rows and 11 columns in size, to a suitable micrograph. The magnification should be such that only a few of the largest crystal are touched by more than one dot and 4000 to 8000 times have proved suitable. At each grid point, if the dot touches a crystal dimension whose shape can be clearly defined, the crystal may be measured. A measure of "scatter", in the form of the Gaussian standard deviation of crystal length with Bessel correction applied is also taken.

Examples 3 to 7, therefore show that when using the compounds of the invention in additive formulations crystals can pass through the filter reliably and the excellent cold temperature performance can be extended to higher fuel wax contents than heretofore practicable and also at temperatures further below fuel Wax Appearance Temperature than heretofore practicable without regard to fuel system considerations such as the ability of recycle fuel from the engine to warm the feed fuel being drawn from the fuel tank, the ratio of feed fuel flow to recycle fuel, the ratio of main filter surface area to feed fuel flow and the size and position of prefilters and screens.

The compound produced in Example 1 was tested for its effectiveness as an additive for distillate fuel in the following Fuels, the boiling characteristic being measured by the ASTM-D86 test.

| | Distillation ASTM-D86 | | | | | | | |
|---|---|---|---|---|---|---|---|---|
| | Cloud Point | Wax Appearance Point | Initial Boiling Point | 20 | 50 | 90 | Final Boiling Point | Untreated CFPP |
| Fuel 1 | -3.0 | -5.0 | 180 | 223 | | 336 | 365 | -5.0 |
| Fuel 2 | +3.0 | +1.0 | 188 | 236 | 278 | 348 | 376 | 0 |
| Fuel 3 | +5.0 | 0.0 | 228 | 280 | 310 | 351 | 374 | -1 |

The results in the Programme Cooling Test carried out at -12°C in Fuel 1 were as follows.

| Additive Used | | | | | Smallest Mesh Passed at | | | Settling | | |
|---|---|---|---|---|---|---|---|---|---|---|
| C | Example 1 | A2 | B | A3 | 50* | 100* | 200* | Wax | Level | % at |
| | | | | | | | | 50* | 100* | 200* |
| 1 | - | - | - | - | 30 | 40 | 80 | 20 | 5 | 2 |
| 4 | - | 1 | - | - | 80 | 100 | 200 | 10 | 5 | 5 |
| 4 | - | - | 1 | - | 40 | 80 | 200 | 15 | 10 | 5 |
| - | 1 | - | - | - | 60 | 120 | 250 | 10 | 100 | 100 |
| - | 4 | 1 | - | - | 150 | 350 | 350 | 50 | 100 | 100 |
| - | 4 | - | 1 | - | 120 | 200 | 250 | 10 | 50 | 100 |
| - | - | 1 | - | - | - | 40 | 60 | - | 10 | 10 |
| - | - | - | - | 1 | - | 60 | 120 | - | 20 | 40 |
| - | - | 1 | - | 3 | - | 60 | 120 | - | 20 | 40 |
| - | - | - | 1 | 4 | - | 80 | 150 | - | 15 | 15 |
| None | | | | | 30 | | | gel | | |

* total ppm concentration of additive.

Further results in Fuel 1 were as follows:

| Additive | Finest PCT Mesh passed at -11°C | |
|---|---|---|
| | 150 | 250 ppm ai |
| A1 | 100 | 200 |
| C | 60 | 120 |
| Example 1 | 200 | 350 |
| Example 2 | 150 | 250 |
| C/A2 (4/1) | 100 | 350 |
| Example 1/A2 (4/1) | 350 | 350 |
| Example 2/A2 (4/1) | 200 | 350 |
| C/B (9/1) | 150 | 250 |
| Example 1/B (9/1) | 250 | 350 |
| C/D (9/1) | 150 | 350 |
| Example 1/D (9/1) | 350 | 350 |
| None | 30 | |

Results in Fuel 3 were as follows:

| Additive | Finest PCT mesh passed at -5°C | | |
|---|---|---|---|
| | 400 ppm | 600 ppm | Wax Level (%) |
| A1 | 40 | 60 | 10 |
| C | 40 | 100 | 10 |
| Example 1 | 80 | 200 | 100 |
| D/C (1/4) | 40 | 120 | 10 |

(continued)

| Additive | Finest PCT mesh passed at -5°C | | |
|---|---|---|---|
| | 400 ppm | 600 ppm | Wax Level (%) |
| D/Example 1 (1/4) | 80 | 250 | 100 |
| None | 20 | | gel |

Further results in Fuel 1 are as follows:

| Additive 1 | Additive 2 | Ratio | Treat Rate | Wax Settling State* % | PCT* at | CFPP (°C) |
|---|---|---|---|---|---|---|
| A2 | | | 100 | 10 | 60 | -11.0 |
| A2 | | | 200 | - | 60 | -14.0 |
| A2 | | | 300 | | 40 | -13.5 |
| A2 | C | 4:1 | 100 | 100 | 30 | -12.0 |
| A2 | C | 4:1 | 200 | 30 | 30 | -11.0 |
| A2 | C | 4:1 | 300 | 20 | 40 | -14.0 |
| A2 | C | 1:1 | 100 | 10 | 60 | -13.5 |
| A2 | C | 1:1 | 200 | 10 | 80 | |
| A2 | C | 1:1 | 300 | 15 | 200 | -18.0 |
| A2 | C | 1:4 | 100 | 10 | 80 | 9.0 |
| A2 | C | 1:4 | 200 | 5 | 100 | -18.5 |
| A2 | C | 1:4 | 300 | 20 | 350 | -18.5 |
| A2 | Ex.1 | 4:1 | 100 | 20 | 40 | -13.0 |
| A2 | Ex.1 | 4:1 | 200 | 20 | 40 | -14.5 |
| A2 | Ex.1 | 4:1 | 300 | 25 | 40 | -15.0 |
| A2 | Ex.1 | 1:1 | 100 | 100 | 150 | -17.0 |
| A2 | Ex.1 | 1:1 | 200 | 100 | 350 | -18.0 |
| A2 | Ex.1 | 1:1 | 300 | 100 | 350 | -19.5 |
| A2 | Ex.1 | 1:4 | 100 | 100 | 350 | -17.0 |
| A2 | Ex.1 | 1:4 | 200 | 100 | 350 | -19.5 |
| A2 | Ex.1 | 1:4 | 300 | 100 | 350 | -19.5 |
| None | None | - | - | gel | 30 | -5.0 |

| Additive 1 | Additive 2 | Ratio | Treat Rate | Wax Settling State* % | PCT* at | CFPP (°C) |
|---|---|---|---|---|---|---|
| A4 | | | 100 | 20 | 60 | -14.0 |
| A4 | | | 200 | 25 | 80 | -16.0 |
| A4 | | | 300 | 30 | 120 | -16.5 |
| A4 | C | 4:1 | 100 | 15 | 60 | -15.0 |
| A4 | C | 4:1 | 200 | 20 | 100 | -16.5 |
| A4 | C | 4:1 | 300 | 20 | 200 | -17.0 |
| A4 | C | 1:1 | 100 | 10 | 80 | -11.0 |
| A4 | C | 1:1 | 200 | 15 | 250 | -17.5 |
| A4 | C | 1:1 | 300 | 15 | 250 | -20.0 |
| A4 | C | 1:4 | 100 | 5 | 100 | -6.5 |
| A4 | C | 1:4 | 200 | 10 | 200 | -16.5 |
| A4 | C | 1:4 | 300 | 10 | 120 | -19.0 |
| A4 | Ex.1 | 4:1 | 100 | 10 | 80 | -13.0 |
| A4 | Ex.1 | 4:1 | 200 | 15 | 100 | -16.5 |
| A4 | Ex.1 | 4:1 | 300 | 20 | 250 | -18.0 |
| A4 | Ex.1 | 1:1 | 100 | 10 | 80 | -17.5 |
| A4 | Ex.1 | 1:1 | 200 | 15 | 80 | -16.0 |
| A4 | Ex.1 | 1:1 | 300 | 100 | 150 | -25.5 |
| A4 | Ex.1 | 1:4 | 100 | 100 | 250 | -19.0 |
| A4 | Ex.1 | 1:4 | 200 | 100 | 350 | -21.0 |
| A4 | Ex.1 | 1:4 | 300 | 100 | 350 | -22.5 |
| None | None | - | - | gel | 30 | -5.0 |

| Additive 1 | Additive 2 | Ratio | Treat Rate | Wax Settling State* % | PCT* at | CFPP (°C) |
|---|---|---|---|---|---|---|
| A1 | | | 100 | 20 | 80 | -16.0 |
| A1 | | | 200 | 40 | 120 | -17.0 |
| A1 | | | 300 | 40 | 200 | -17.5 |
| A1 | C | 4:1 | 100 | 15 | 100 | -15.0 |
| A1 | C | 4:1 | 200 | 30 | 200 | -19.0 |
| A1 | C | 4:1 | 300 | 40 | 250 | -19.5 |
| A1 | C | 1:1 | 100 | 10 | 150 | -14.5 |
| A1 | C | 1:1 | 200 | 30 | 200 | -19.5 |
| A1 | C | 1:1 | 300 | 25 | 250 | -22.0 |
| A1 | C | 1:4 | 100 | 20 | 150 | -15.0 |
| A1 | C | 1:4 | 200 | 100 | 150 | -19.0 |
| A1 | C | 1:4 | 300 | 100 | 350 | -20.0 |
| A1 | Ex.1 | 4:1 | 100 | 20 | 120 | -14.5 |
| A1 | Ex.1 | 4:1 | 200 | 100 | 150 | -19.5 |
| A1 | Ex.1 | 4:1 | 300 | 100 | 200 | -24.5 |
| A1 | Ex.1 | 1:1 | 100 | 10 | 150 | -18.0 |
| A1 | Ex.1 | 1:1 | 200 | 25 | 200 | -24.5 |
| A1 | Ex.1 | 1:1 | 300 | 25 | 250 | -25.0 |
| A1 | Ex.1 | 1:4 | 100 | 100 | 150 | -18.5 |
| A1 | Ex.1 | 1:4 | 200 | 100 | 350 | -20.5 |
| A1 | Ex.1 | 1:4 | 300 | 100 | 350 | -24.5 |
| None | None | - | - | gel | 30 | -5.0 |

*at -12°C

| Wax Settling | | | | | | |
|---|---|---|---|---|---|---|
| Additive | | Ratio | Treat Rate | State* % | PCT* at | CFPP (°C) |
| 1 | 2 | | | | | |
| A3 | | | 100 | 20 | 80 | -9.5 |
| A3 | | | 200 | 40 | 150 | -14.0 |
| A3 | | | 300 | 70 | 200 | -19.0 |
| A3 | C | 4:1 | 100 | 10 | 100 | -6.5 |
| A3 | C | 4:1 | 200 | 20 | 200 | -11.0 |
| A3 | C | 4:1 | 300 | 25 | 250 | -16.0 |
| A3 | C | 1:1 | 100 | 5 | 80 | -5.0 |
| A3 | C | 1:1 | 200 | 15 | 200 | -6.5 |
| A3 | C | 1:1 | 300 | 30 | 250 | -7.5 |
| A3 | C | 1:4 | 100 | 10 | 40 | -4.5 |
| A3 | C | 1:4 | 200 | 10 | 120 | -4.5 |
| A3 | C | 1:4 | 300 | 15 | 150 | -5.5 |
| A3 | Ex.1 | 4:1 | 100 | 10 | 100 | -6.0 |
| A3 | Ex.1 | 4:1 | 200 | 20 | 150 | -9.5 |
| A3 | Ex.1 | 4:1 | 300 | 25 | 200 | -16.0 |
| A3 | Ex.1 | 1:1 | 100 | 5 | 80 | -5.5 |
| A3 | Ex.1 | 1:1 | 200 | 20 | 150 | -8.0 |
| A3 | Ex.1 | 1:1 | 300 | 20 | 120 | -16.5 |
| A3 | Ex.1 | 1:4 | 100 | 5 | 40 | -4.0 |
| A3 | Ex.1 | 1:4 | 200 | 10 | 60 | -6.5 |
| A3 | Ex.1 | 1:4 | 300 | 25 | 150 | -9.0 |
| None | None | - | - | gel | 30 | -5.0 |

* at -12°C

Results in Fuel 2 were as follows

| Wax Settling | | | | | | |
|---|---|---|---|---|---|---|
| Additive | | Ratio | Treat Rate | State* % | PCT* at | CFPP (°C) |
| 1 | 2 | | | | | |
| A2 | | | 300 | 25 | 30 | -4.5 |
| A2 | | | 500 | 10 | 40 | -9.5 |
| A2 | | | 700 | 15 | 60 | -11.0 |
| A2 | C | 4:1 | 300 | 15 | 60 | -11.0 |
| A2 | C | 4:1 | 500 | 15 | 60 | -12.0 |
| A2 | C | 4:1 | 700 | 15 | 40 | -15.0 |
| A2 | C | 1:1 | 300 | 20 | 40 | -15.0 |
| A2 | C | 1:1 | 500 | 10 | 80 | -16.0 |
| A2 | C | 1:1 | 700 | 20 | 200 | -15.5 |
| A2 | C | 1:4 | 300 | 10 | 100 | -12.5 |
| A2 | C | 1:4 | 500 | 10 | 200 | -14.0 |
| A2 | C | 1:4 | 700 | 100 | 250 | -15.0 |
| A2 | C | 4:1 | 300 | 15 | 40 | -7.5 |

* at -7°C

(continued)

| Wax Settling | | | | | | |
|---|---|---|---|---|---|---|
| Additive | | Ratio | Treat Rate | State* % | PCT* at | CFPP (°C) |
| 1 | 2 | | | | | |
| A2 | C | 4:1 | 500 | 15 | 60 | -17.0 |
| A2 | C | 4:1 | 700 | 20 | 100 | -19.0 |
| A2 | Ex.1 | 1:1 | 300 | 10 | 80 | -14.5 |
| A2 | Ex.1 | 1:1 | 500 | 15 | 120 | -18.0 |
| A2 | Ex.1 | 1:1 | 700 | 20 | 150 | -18.0 |
| A2 | Ex.1 | 1:4 | 300 | 100 | 150 | -18.0 |
| A2 | Ex.1 | 1:4 | 500 | 100 | 350 | -15.0 |
| A2 | Ex.1 | 1:4 | 700 | 100 | 350 | -20.0 |
| None | None | - | - | gel | 30 | 0.0 |

* at -7°C

| Wax Settling | | | | | | |
|---|---|---|---|---|---|---|
| Additive | | Ratio | Treat Rate | State* % | PCT* at | CFPP (°C) |
| 1 | 2 | | | | | |
| A4 | | | 300 | 30 | 100 | -15.5 |
| A4 | | | 500 | 35 | 120 | -15.5 |
| A4 | | | 700 | 45 | 120 | -16.5 |
| A4 | C | 4:1 | 300 | 20 | 100 | -15.0 |
| A4 | C | 4:1 | 500 | 20 | 120 | -15.0 |
| A4 | C | 4:1 | 700 | 25 | 150 | -15.0 |
| A4 | C | 1:1 | 300 | 10 | 100 | -15.0 |
| A4 | C | 1:1 | 500 | 25 | 150 | -17.5 |
| A4 | C | 1:1 | 700 | 20 | 200 | -18.5 |
| A4 | C | 1:4 | 300 | 10 | 120 | -11.5 |
| A4 | C | 1:4 | 500 | 15 | 200 | -15.5 |
| A4 | C | 1:4 | 700 | 20 | 250 | -14.5 |
| A4 | Ex.1 | 4:1 | 300 | 15 | 100 | -17.0 |
| A4 | Ex.1 | 4:1 | 500 | 25 | 120 | -17.0 |
| A4 | Ex.1 | 4:1 | 700 | 25 | 150 | -19.5 |
| A4 | Ex.1 | 1:1 | 300 | 10 | 120 | -13.5 |
| A4 | Ex.1 | 1:1 | 500 | 15 | 200 | -19.0 |
| A4 | Ex.1 | 1:1 | 700 | 20 | 250 | -21.0 |
| A4 | Ex.1 | 1:4 | 300 | 100 | 150 | |
| A4 | Ex.1 | 1:4 | 500 | 100 | 350 | |
| A4 | Ex.1 | 1:4 | 700 | 100 | 350 | -18.5 |
| None | None | - | - | gel | 30 | 0.0 |

* at -7°C

| Wax Settling | | | | | | |
|---|---|---|---|---|---|---|
| Additive | | Ratio | Treat Rate | State* % | PCT* at | CFPP (°C) |
| 1 | 2 | | | | | |
| A1 | | | 300 | 40 | 120 | -15.0 |
| A1 | | | 500 | 40 | 150 | -19.0 |
| A1 | | | 700 | 60 | 200 | -18.5 |
| A1 | C | 4:1 | 300 | 25 | 120 | -15.0 |
| A1 | C | 4:1 | 500 | 30 | 150 | -14.0 |
| A1 | C | 4:1 | 700 | 50 | 200 | -17.0 |
| A1 | C | 1:1 | 300 | 15 | 120 | -17.0 |
| A1 | C | 1:1 | 500 | 25 | 150 | -16.0 |
| A1 | C | 1:1 | 700 | 25 | 200 | -19.0 |
| A1 | C | 1:4 | 300 | 15 | 80 | -13.0 |
| A1 | C | 1:4 | 500 | 15 | 120 | -13.0 |
| A1 | C | 1:4 | 700 | 15 | 250 | -15.0 |
| A1 | Ex.1 | 4:1 | 300 | 20 | 120 | -18.0 |
| A1 | Ex.1 | 4:1 | 500 | 30 | 200 | -16.5 |
| A1 | Ex.1 | 4:1 | 700 | 40 | 250 | -15.0 |
| A1 | Ex.1 | 1:1 | 300 | 15 | 120 | -15.5 |
| A1 | Ex.1 | 1:1 | 500 | 25 | 250 | -20.0 |
| A1 | Ex.1 | 1:1 | 700 | 25 | 350 | -19.5 |
| A1 | Ex.1 | 1:4 | 300 | 100 | 150 | -16.5 |
| A1 | Ex.1 | 1:4 | 500 | 100 | 350 | -20.0 |
| A1 | Ex.1 | 1:4 | 700 | 100 | 350 | -19.0 |
| None | None | - | - | gel | 30 | 0.0 |

| Wax Settling | | | | | | |
|---|---|---|---|---|---|---|
| Additive | | Ratio | Treat Rate | State* % | PCT* at | CFPP (°C) |
| 1 | 2 | | | | | |
| A3 | | | 300 | 40 | 100 | -13.5 |
| A3 | | | 500 | 40 | 100 | |
| A3 | | | 700 | 40 | 150 | |
| A3 | C | 4:1 | 300 | 30 | 150 | -14.5 |
| A3 | C | 4:1 | 500 | 40 | 150 | -17.0 |
| A3 | C | 4:1 | 700 | 50 | 200 | -15.5 |
| A3 | C | 1:1 | 300 | 30 | 150 | -9.5 |
| A3 | C | 1:1 | 500 | 40 | 200 | -14.5 |
| A3 | C | 1:1 | 700 | 40 | 250 | -12.5 |
| A3 | C | 1:4 | 300 | 20 | 100 | -3.0 |
| A3 | C | 1:4 | 500 | 20 | 120 | -7.0 |
| A3 | C | 1:4 | 700 | 20 | 250 | -12.0 |
| A3 | Ex.1 | 4:1 | 300 | 40 | 120 | -13.0 |
| A3 | Ex.1 | 4:1 | 500 | 40 | 150 | -15.0 |
| A3 | Ex.1 | 4:1 | 700 | 50 | 200 | -15.0 |

*at -7°C

(continued)

| Wax Settling | | | | | | |
|---|---|---|---|---|---|---|
| Additive | | Ratio | Treat Rate | State* % | PCT* at | CFPP (°C) |
| 1 | 2 | | | | | |
| A3 | Ex.1 | 1:1 | 300 | 30 | 150 | -7.0 |
| A3 | Ex.1 | 1:1 | 500 | 40 | 200 | -13.5 |
| A3 | Ex.1 | 1:1 | 700 | 60 | 250 | -14.0 |
| A3 | Ex.1 | 1:4 | 300 | 20 | 150 | -6.5 |
| A3 | Ex.1 | 1:4 | 500 | 100 | 350 | -13.5 |
| A3 | Ex.1 | 1:4 | 700 | 100 | 350 | -17.0 |
| None | None | - | - | gel | 30 | 0.0 |

*at -7°C

Example 9

The fuel used in this Example had the following characteristics:

| (ASTM-D86) | |
|---|---|
| IBP | 190°C |
| 20% | 246°C |
| 90% | 346°C |
| FBP | 374°C |
| Wax Appearance Temperature | -15°C |
| Cloud Point | +2.0°C |

It was treated with 1000 parts per million of active ingredient of the following additives:

(E) A mixture of Additive A2 (1 part by weight) and Additive C (9 parts by weight).

(F) The commercial ethylene vinyl acetate copolymer additive marketed by Exxon Chemicals as ECA 5920.

(G) A mixture of:

1 part Additive of Example 1
1 part Additive E1
1 part Additive D
1 part of Additive K

(H) The commercial ethylene vinyl acetate copolymer additive marketed by Amoco as 2042E.

(I) The commercial ethylene vinyl propionate copolymer additive marketed by BASF as Keroflux 5486.

(J) No additive.

(K) The reaction product of 4 moles of dihydrogenated-tallow amine and 1 mole of pyromellitic anhydride. The reaction is performed solventless at 150oC, stirring under nitrogen for 6 hours.

The following performance characteristics of these fuels were then measured.

(i) The ability of the fuel to pass through the diesel fuel main filter at -9°C, and the percentage of wax passing through the filter with the following results:

| Additive | Time to Failure | % of Wax Passing |
|---|---|---|
| E | 11 minutes | 18-30% |
| F | 16 minutes | 30% |
| G | No Failure | 90-100% |
| H | 15 minutes | 25% |
| I | 12 minutes | 25% |
| J | 9 minutes | 10% |

(ii) The pressure drop across the main filter against time is indicative of any wax passing through the filter and the results are shown graphically in Figure 9 with the following results:

(iii) The wax settling in the fuels was measured by cooling 100 mls of fuel in a graduated measuring cylinder. The cylinder is cooled at 1°C/hour from a temperature preferably 10°C above the fuel's Cloud Point but not less than 5°C above the fuel's Cloud Point to the test temperature, which is then held for a prescribed time. The test temperature and soak time depend upon the application, i.e. diesel fuel and heating oil. It is preferred that the test temperature be at least 5°C below the Cloud Point and the minimum cold soak time at the test temperature be at least 4 hours. Preferably the test temperature should be 10°C or more below the fuel's Cloud Point and the soak period should be 24 hours or more.

After the end of the soak period the measuring cylinder is examined and the extent of wax crystal settling is visually measured as the height of any wax layer above the bottom of the cylinder (0 mls) and expressed in terms of a percentage of the total volume (100 mls). Clear fuel may be seen above the settled wax crystals and this form of measurement is often sufficient to form a judgement on the wax settling.

Sometimes the fuel is cloudy above a settled wax crystal layer or the wax crystals can be seen to be visibly denser as they approach the bottom of the cylinder. In this case a more quantitative method of analysis is used. Here the top 5% (5 mls) of fuel is sucked off carefully and stored, the next 45% is sucked off and discarded, the next 5% is sucked off and stored, the next 35% is sucked off and discarded and finally the bottom 10% is collected after warming to dissolve the wax crystals. These stored samples will henceforth be referred to as Top, Middle and Bottom samples respectively. It is important that the vacuum applied to remove the samples be fairly low, i.e. 200 mm water pressure, and that the top of the pipette is placed just on the surface of the fuel to avoid currents in the liquid which could disturb the concentration of wax at different layers within the cylinder. The samples are then warmed to 60ºC for 15 minutes and examined for wax content by Differential Scanning Calorimetry (DSC) as previously described.

In this instance a Mettler TA 2000B DSC machine was used. A 25 ul sample is placed in the sample cell and regular kerosine in the reference cell then they are cooled at 22°C/minute from 60°C to at least 10°C but preferably 20°C above the Wax Appearance Temperature (WAT) then it is cooled at 2°C/minute to approximately 20°C below the WAT. A reference must be run of the unsettled, uncooled treated fuel. The extent of wax settling then correlates with the WAT (or Δ WAT = WAT settled sample - AT original). Negative values indicate dewaxing of the fuel and positive values indicate wax enrichment through settling. The wax content may also be used as a measure of settling from these samples. This is illustrated by % WAX or Δ% WAX (Δ%Wax = % Wax settled sample - % Wax original) and, once again negative values indicate dewaxing of the fuel and positive values indicate wax enrichment through settling.

In this example the fuel was cooled at 1°C/hr from +10°C down to -9°C and cold soaked for 48 hours prior to testing. The results were as follows:

| Additive | Visual Wax Settling | WAT °C Data Settled Samples | | |
|---|---|---|---|---|
| | | Top 5% | Middle 5% | Bottom 10% |
| E | Cloudy throughout Denser at bottom | -10.80 | -4.00 | -3.15 |
| F | 50% clear above | -13.35 | -0.80 | -0.40 |
| G | 100% | -7.85 | -7.40 | -7.50 |
| H | 35% clear above | -13.05 | -8.50 | +0.50 |
| I | 65% clear above | | | |
| J | 100% Semi-gel | -6.20 | -6.25 | -6.40 |

(The results are also shown graphically in Figure 10).

| WAT original | | Δ WAT (°C) (Settled Samples) | | |
|---|---|---|---|---|
| (Unsettled Fuel) | | Top 5% | Middle 5% | Bottom 10% |
| E | -6.00 | -4.80 | +2.00 | +2.85 |
| F | -5.15 | -8.20 | +4.35 | +4.75 |
| G | -7.75 | -0.10 | +0.35 | +0.25 |
| H | -5.00 | -8.05 | -3.50 | +4.50 |
| J | -6.20 | 0.00 | -0.05 | -0.20 |
| Note that significant depression of the WAT can be achieved by the most effective additive (G)) | | | | |

| | Δ % WAX (Settled Samples) | | |
|---|---|---|---|
| | Top 5% | Middle 5% | Bottom 10% |
| E | -7.0 | +0.8 | +0.9 |
| F | -0.8 | +2.1 | +2.2 |
| G | ±0.0 | +0.3 | +0.1 |
| H | -1.3 | -0.2 | +1.1 |
| J | -0.1 | ±0.0 | ±0.1 |

These results show that as the crystal size is reduced by the presence of additives, the wax crystals settle relatively quickly. For example, untreated fuels when cooled below their cloud points tend to show little wax crystal settling because the plate-like crystals interlock and cannot tumble freely in the liquid and a gel-like structure is set up but when a flow improver is added the crystals may be modified so their habit becomes less plate-like and tends to form needles of sizes in the range of tens of micrometers which can move freely in the liquid and settle relatively rapidly. This wax crystal settling can cause problems in storage tanks and vehicle systems. Concentrated wax layers may be unexpectedly drawn off, especially when the fuel level is low or the tank disturbed (e.g. when a vehicle corners), and filter blockage may occur.

If the wax crystal size can be reduced still further to below 10000 nanometres then the crystals settle relatively slowly and Wax Anti Settling can result giving the benefits in fuel performance compared to the case of a fuel with settled wax crystals. If the wax crystal size can be reduced to below approximately 4000 nanometres then the tendency of the crystals to settle is almost eliminated within the time of fuel storage. If the crystal sizes are reduced to the preferred size of below 2000 nanometres claim then the wax crystals remain suspended in the fuel for the many weeks required in some storage systems and the problems of settling are substantially eliminated.

(iv) The CFPP performance which was as follows:

| Additive | CFPP Temperature (°C) | CFPP Depression |
|---|---|---|
| E | -14 | 11 |
| F | -20 | 17 |
| G | -20 | 17 |
| H | -20 | 17 |
| I | -19 | 16 |
| J | -3 | - |

(v) The average crystal size which was found to be:

| Additive | Size |
|---|---|
| | (Nanometers) |
| E | 4400 |
| F | 10400 |
| G | 2600 |

(continued)

| Additive | Size |
|---|---|
| | (Nanometers) |
| H | 10000 |
| I | 8400 |
| J | Thin plates in excess of 50000 |

## Claims

1. Distillate fuel boiling in the range 120° C to 500° C containing 0.001 to 0.5 wt% of a compound of the general formula

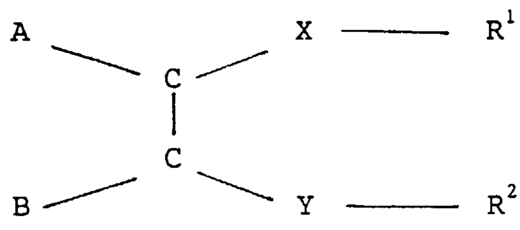

in which

-Y-$R^2$ is -$SO_3$(-)(+)$NR^3{}_3R^2$, -$SO_3$(-)(+)$HNR^3{}_2R^2$, -$SO_3$(-)(+)$H_2NR^3R^2$, -$SO_3$(-)(+)$H_3NR^2$, -$SO_2NR^3R^2$ or -$SO_3R^2$;
-X-$R^1$ is -Y-$R^2$ or -$CONR^3R^1$, -$CO_2$(-)(+)$NR^3{}_3R^1$, -$CO_2$(-)(+)$HNR^3{}_2R^1$, -$CO_2$(-)(+)$H_2NR^3R^1$, -$CO_2$(-)(+)$H_3NR^1$, -$R^4$-$COOR_1$, -$NR^3COR^1$, -$R^4OR^1$, -$R^4OCOR^1$, -$R^4R^1$ -N($COR^3$)$R^1$ or Z(-)(+)$NR^3{}_3R^1$;
-Z(-) is $SO_3$(-) or -$CO_2$(-);

$R^1$ and $R^2$ are alkyl, alkoxy alkyl or polyalkoxy alkyl containing at least 10 carbon atoms in the main chain;

$R^3$ is hydrocarbyl and each $R^3$ may be the same or different and

$R^4$ is nothing or is $C_1$ to $C_5$ alkylene and in

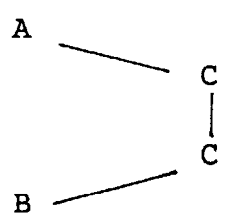

the carbon-carbon (C-C) bond is either

a) ethylenically unsaturated when A and B may be alkyl, alkenyl or substituted hydrocarbyl groups or

b) part of a cyclic structure which may be aromatic, polynuclear aromatic or cycloaliphatic,

with the proviso that if X-$R^1$ and/or Y-$R^2$ are -$SO_3$(-)(+)$H_3NR^2$ the distillate fuel does not contain a copolymer of (1) an alpha-olefin having two to seventeen carbon atoms per molecule or an aromatic substituted olefin having eight to forty carbon atoms per molecule and (2) an ester, said ester being a mono- or di-alkyl fumarate, itaconate, citraconate, mesaconate, trans- or cis-glutaconate, in which the alkyl group has 8 to 23 carbon atoms.

2. Distillate fuel according to Claim 1 in which the groups $R^1$, $R^2$ and $R^3$ are alkyl groups.

3. Distillate fuel according to Claim 1 or 2 in which the groups $R^1$, $R^2$ and $R^3$ are straight chain alkyl groups containing at least 10 carbon atoms.

4. Distillate fuel according to any of claims 1 to 3 in which the additive is the N,N-dialkyl ammonium salt of 2-(dialkylamido)-benzene sulphonic acid or 2-(alkylcarboxy)-benzene sulphonic acid.

5. Distillate fuel according to any of Claims 1 to 4 together with other distillate fuel additives.

6. Distillate fuel according to Claim 5 in which one of the other fuel additives is a copolymer of ethylene and an ethylenically unsaturated ester.

7. Distillate fuel according to Claim 5 or 6 in which one of the other fuel additives is an esterified copolymer of styrene and maleic anhydride.

8. Distillate fuel according to any of Claims 5 to 7 in which one of the other fuel additives is an olefine copolymer.

9. Distillate fuel according to Claim 8 in which the olefine copolymer is a copolymer of ethylene and a $C_3$ to $C_6$ olefine.

## Patentansprüche

1. Destillatbrennstoff, der im Bereich von 120 °C bis 500 °C siedet und 0,001 bis 0,5 Gew.% einer Verbindung der allgemeinen Formel

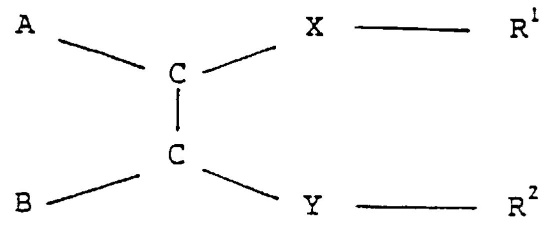

enthält, in der

-Y-$R^2$ $-SO_3^{(-)(+)}NR^3_3R^2$, $-SO_3^{(-)(+)}HNR^3_2R^2$, $-SO_3^{(-)(+)}H_2NR^3R^2$, $-SO_3^{(-)(+)}H_3NR^2$, $-SO_2NR^3R^2$ oder $-SO_3R^2$ ist;
-X-$R^1$ -Y-$R^2$ oder $-CONR^3R^1$, $-CO_2^{(-)(+)}NR^3_3R^1$, $-CO_2^{(-)(+)}HNR^3_2R^1$, $-CO_2^{(-)(+)}H_2NR^3R^1$, $-CO_2^{(-)(+)}H_3NR^1$, $-R^4-COOR_1$, $-NR^3COR^1$, $-R^4OR^1$, $-R^4OCOR^1$, $-R^4R^1$, $-N(COR^3)R^1$ oder $Z^{(-)(+)}NR^3_3R^1$ ist; $-Z^{(-)}$ $SO_3^{(-)}$ oder $-CO_2^{(-)}$ ist;

$R^1$ und $R^2$ Alkyl-, Alkoxyalkyl- oder Polyalkoxyalkylgruppen mit mindestens 10 Kohlenstoffatomen in der Hauptkette sind,

$R^3$ Kohlenwasserstoff ist, wobei jedes $R^3$ gleich oder verschieden sein kann,

$R^4$ ohne Bedeutung ist oder $C_1$- bis $C_5$-Alkylen ist und in

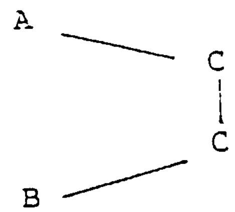

die Kohlenstoff-Kohlenstoff-(C-C)-Bindung entweder

a) ethylenisch ungesättigt ist, wenn A und B Alkyl-, Alkenyl- oder substituierte Kohlenwasserstoffgruppen sein können, oder

b) Teil einer cyclischen Struktur ist, die aromatisch, mehrkernig aromatisch oder cycloaliphatisch sein kann,

mit der Maßgabe, daß, wenn X-$R^1$ und/oder Y-$R^2$ -$SO_3^{(-)(+)}H_3NR^2$ sind, der Destillatbrennstoff kein Copolymer aus (1) einem α-Olefin mit zwei bis siebzehn Kohlenstoffatomen pro Molekül oder einem aromatisch substituierten Olefin mit acht bis vierzig Kohlenstoffatomen pro Molekül und (2) einem Ester enthält, wobei der Ester ein Mono- oder Dialkylfumarat, -itakonat, -citrakonat, -mesakonat oder trans- oder cisglutaconat ist, in dem die Alkylgruppe 8 bis 23 Kohlenstoffatome aufweist.

**2.** Destillatbrennstoff nach Anspruch 1, bei dem die Gruppen $R^1$, $R^2$ und $R^3$ Alkylgruppen sind.

**3.** Destillatbrennstoff nach Anspruch 1 oder Anspruch 2, bei dem die Gruppen $R^1$, $R^2$ und $R^3$ geradkettige Alkylgruppen mit mindestens 10 Kohlenstoffatomen sind.

**4.** Destillatbrennstoff nach einem der Ansprüche 1 bis 3, bei dem das Additiv das N,N-Dialkylammoniumsalz von 2-(Dialkylamido)benzolsulfonsäure oder 2-(Alkylcarboxy)benzolsulfonsäure ist.

**5.** Destillatbrennstoff nach einem der Ansprüche 1 bis 4 zusammen mit anderen Destillatbrennstoffadditiven.

**6.** Destillatbrennstoff nach Anspruch 5, bei dem eines der anderen Brennstoffadditive ein Copolymer von Ethylen und einem ethylenisch ungesättigten Ester ist.

**7.** Destillatbrennstoff nach Anspruch 5 oder 6, bei dem eines der anderen Brennstoffadditive ein verestertes Copolymer von Styrol und Maleinsäureanhydrid ist.

**8.** Destillatbrennstoff nach einem der Ansprüche 5 bis 7, bei dem eines der anderen Brennstoffadditive ein Olefincopolymer ist.

**9.** Destillatbrennstoff nach Anspruch 8, bei dem das Olefincopolymer ein Copolymer von Ethylen und einem $C_3$- bis $C_6$-Olefin ist.

## Revendications

**1.** Combustible distillé bouillant dans la plage de 120°C à 500°C, contenant 0,001 à 0,5 % en poids d'un composé de formule générale

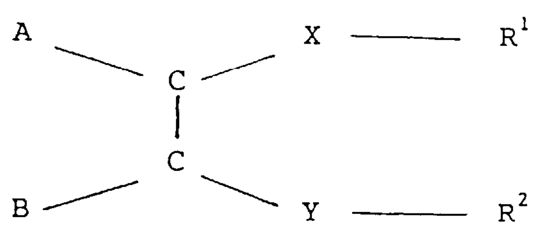

dans laquelle

-Y-$R^2$ représente un groupe -$SO_3$(-) (+) $NR^3_3R^2$, -$SO_3$ (-) (+) $HNR^3_2R^2$, -$SO_3$(-) (+) $H_2NR^3R^2$, -$SO_3$(-) (+) $H_3NR^2$, -$SO_2NR^3R^2$ ou -$SO_3R^2$;
-X-$R^1$ représente un groupe -Y-$R^2$ ou -$CONR^3R^1$, -$CO_2$(-) (+) $NR^3_3R^1$, -$CO_2$(-)(+) $HNR^3_2R^1$, -$CO_2$(-) (+) $H_2NR^3R^1$, -$CO_2$ (-) (+) $H_3NR^1$, -$R^4$-$COOR_1$, -$NR^3COR^1$, -$R^4OR^1$, -$R^4OCOR^1$, -$R^4R^1$, -N($COR^3$)$R^1$ ou Z(-) (+)

$NR^3{}_3R^1$ ;
-Z(-) représente un groupe $SO_3$(-) ou -$CO_2$(-) ;

$R^1$ et $R^2$ représentent des groupes alkyle, alkoxy-alkyle ou polyalkoxyalkyle contenant au moins 10 atomes de carbone dans la chaîne principale ;
$R^3$ représente un groupe hydrocarbyle, les groupes $R^3$ pouvant être identiques ou différents, et
$R^4$ est absent ou bien représente un groupe alkylène en $C_1$ à $C_5$ et, dans le groupe

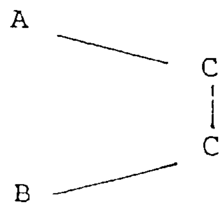

la liaison carbone-carbone (C-C)

a) est une liaison à insaturation éthylénique dans laquelle A et B peuvent représenter des groupes alkyle, des groupes alcényle ou des groupes hydrocarbyle substitués, ou bien
b) fait partie d'une structure cyclique qui peut être aromatique, aromatique polycyclique ou cycloaliphatique,

sous réserve que, si X-$R^1$ e/ou Y-$R^2$ représentent des groupes -$SO_3$(-) (+)$H_3NR^2$, le combustible distillé ne contienne pas un copolymère (1) d'une alpha-oléfine ayant deux à dix-sept atomes de carbone par molécule ou d'une oléfine à substituant aromatique ayant huit à quarante atomes de carbone par molécule, et (2) d'un ester, ledit ester étant un fumarate, itaconate, citraconate, mésaconate, trans- ou cis-glutaconate de mono- ou dialkyle, dans lequel le groupe alkyle a 8 à 23 atomes de carbone.

**2.** Combustible distillé suivant la revendication 1, dans lequel les groupes $R^1$, $R^2$ et $R^3$ représentent des groupes alkyle.

**3.** Combustible distillé suivant la revendication 1 ou 2, dans lequel les groupes $R^1$, $R^2$ et $R^3$ représentent des groupes alkyle à chaîne droite contenant au moins 10 atomes de carbone.

**4.** Combustible distillé suivant l'une quelconque des revendications 1 à 3, dans lequel l'additif est un sel de N,N-dialkylammonium d'acide 2-(dialkylamido)benzènesulfonique ou d'acide 2-(alkylcarboxy)benzènesulfonique.

**5.** Combustible distillé suivant l'une quelconque des revendications 1 à 4, en association avec d'autres additifs pour combustibles distillés.

**6.** Combustible distillé suivant la revendication 5, dans lequel un des autres additifs pour combustibles est un copolymère d'éthylène et d'un ester à insaturation éthylénique.

**7.** Combustible distillé suivant la revendication 5 ou 6, dans lequel un des autres additifs pour combustibles est un copolymère estérifié de styrène et d'anhydride maléique.

**8.** Combustible distillé suivant l'une quelconque des revendications 5 à 7, dans lequel un des autres additifs pour combustibles est un copolymère d'oléfine.

**9.** Combustible distillé suivant la revendication 8, dans lequel le copolymère d'oléfine est un copolymère d'éthylène et d'une oléfine en $C_3$ à $C_6$.

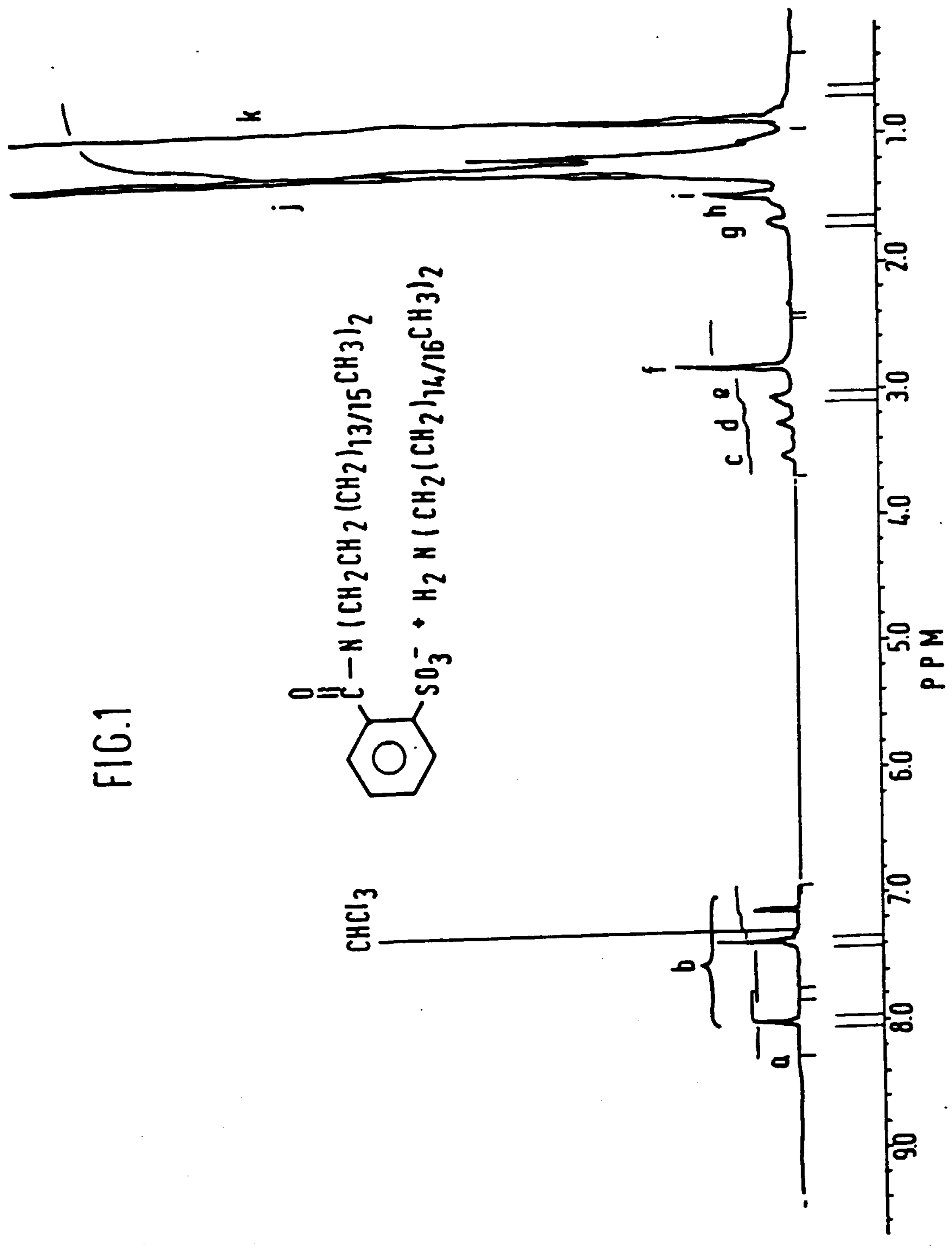


FIG.1

FIG.2

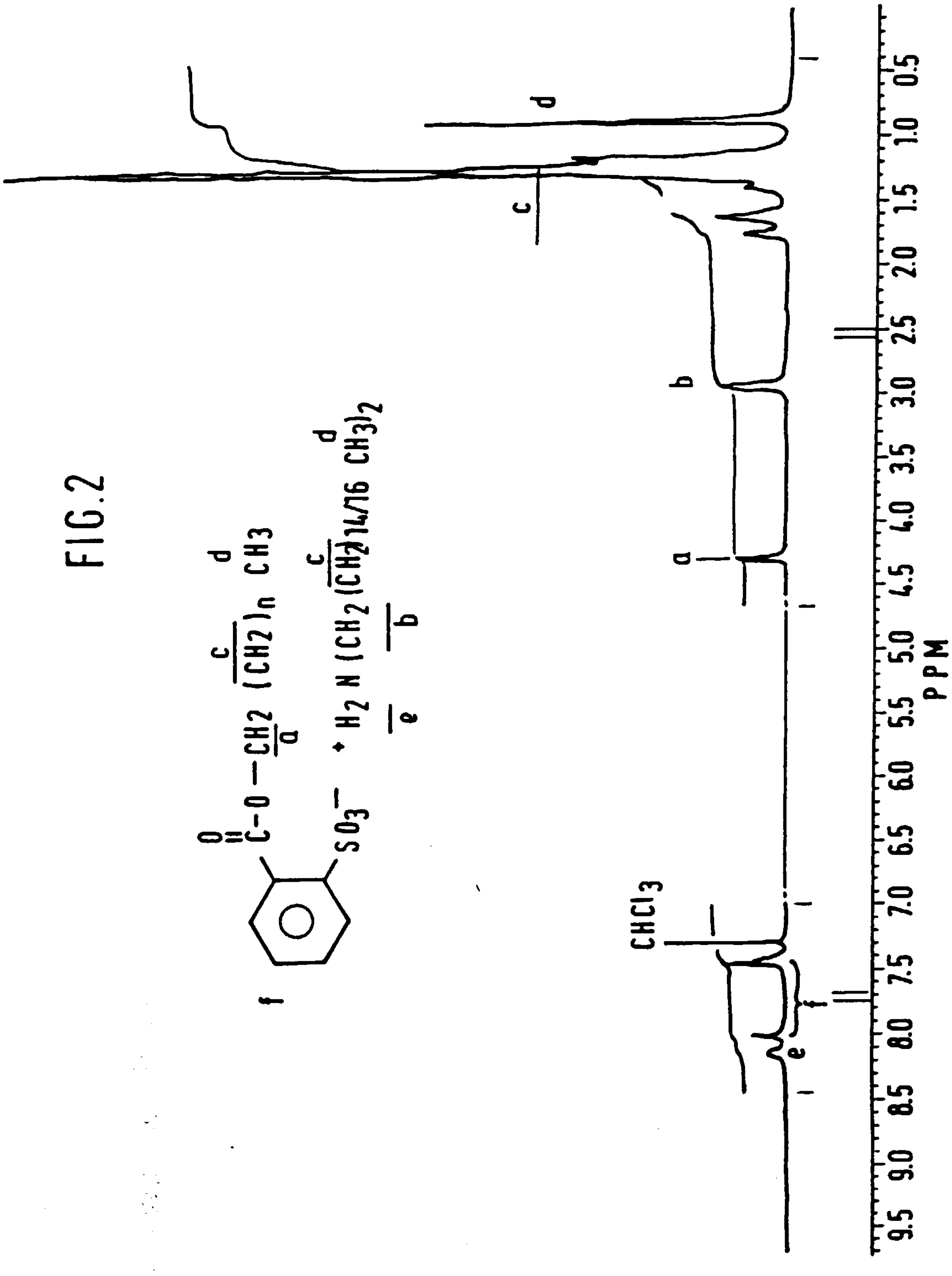

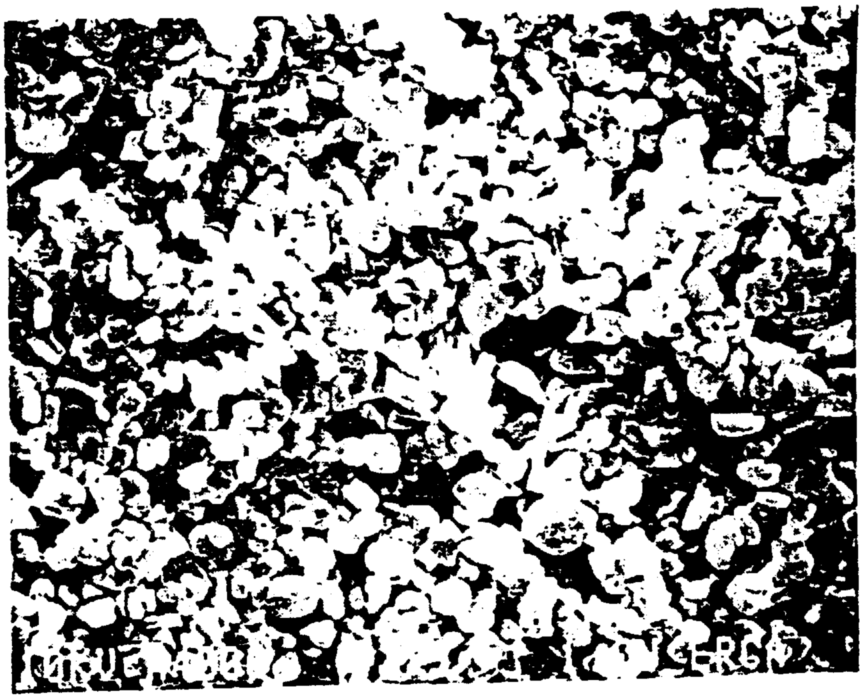

FIG. 3

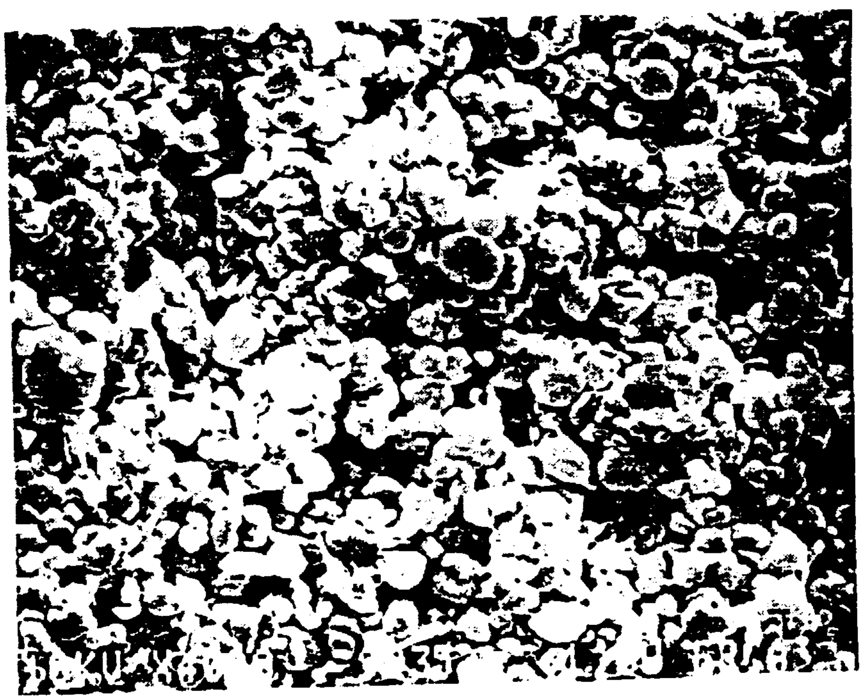

FIG. 4

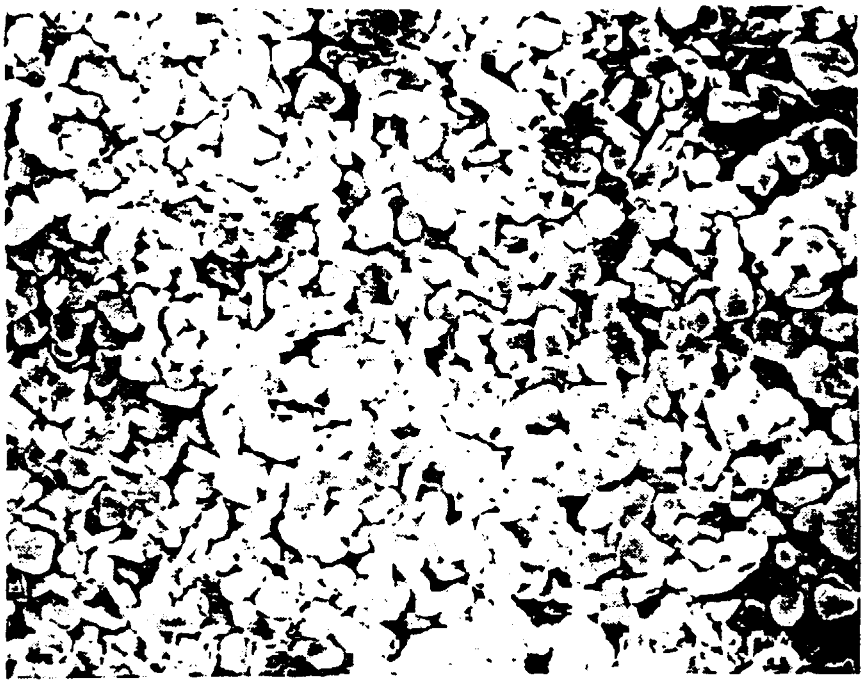

FIG.5

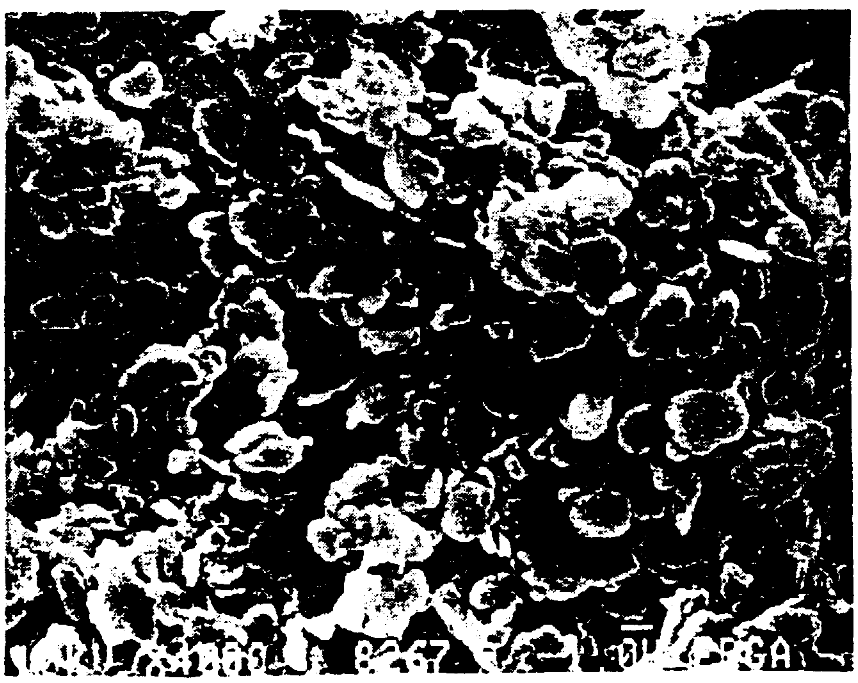

FIG.6

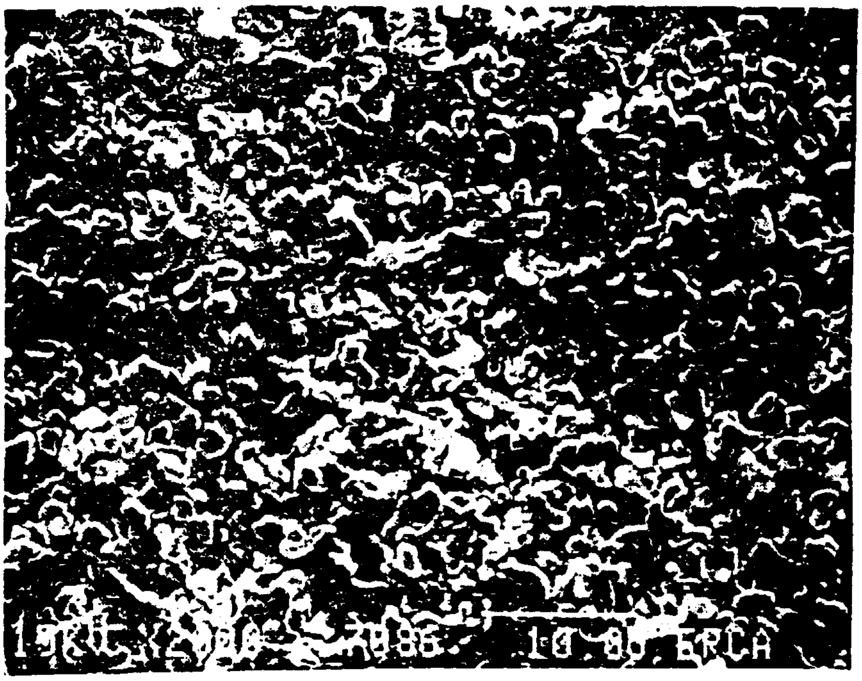


FIG.7

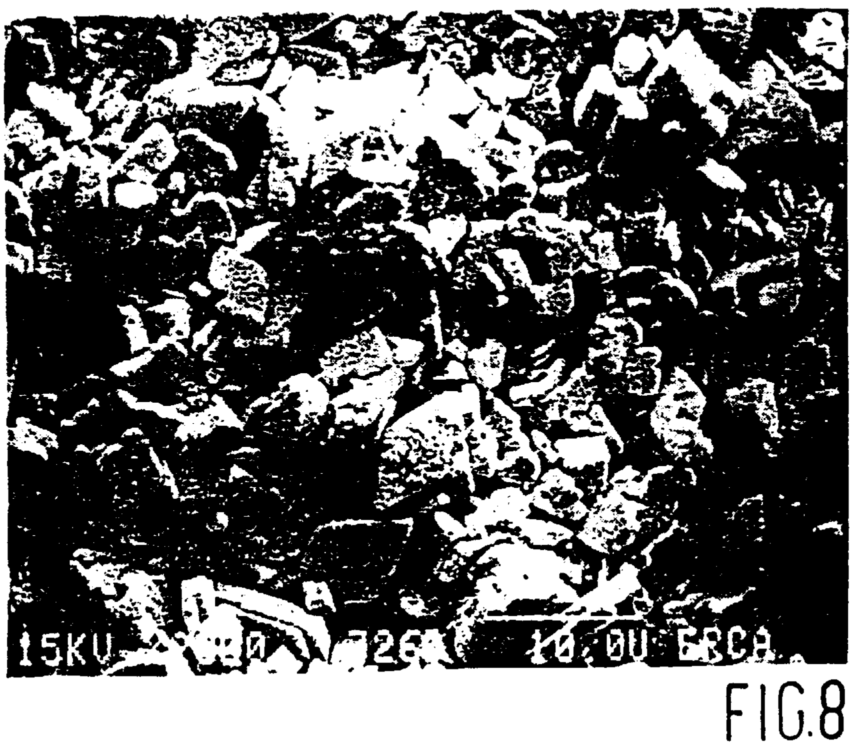


FIG.8

FIG. 9

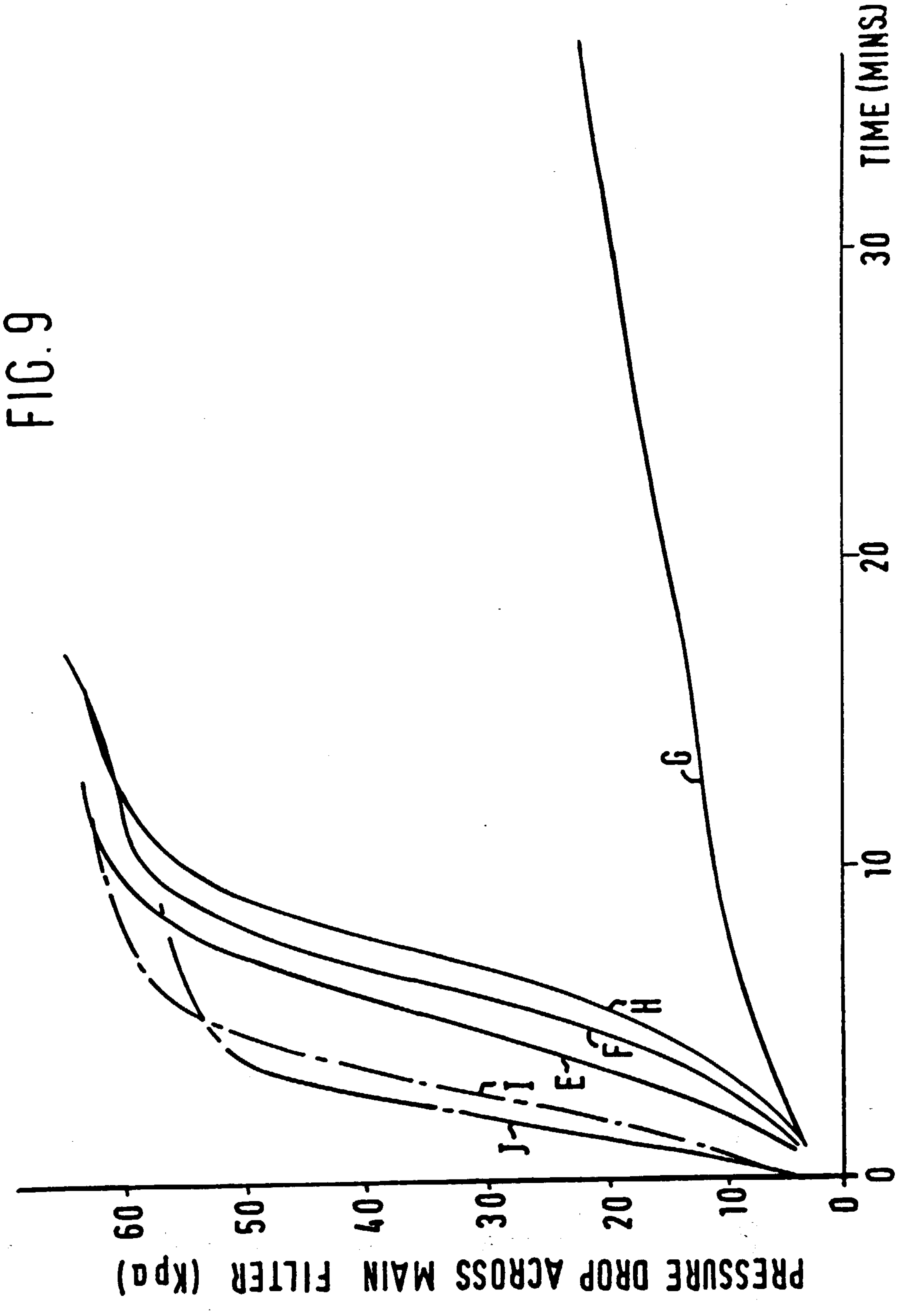
PRESSURE DROP ACROSS MAIN FILTER (Kpa)
0
10
20
30
40
50
60
TIME (MINS)
0
10
20
30
J
I
E
F
H
G

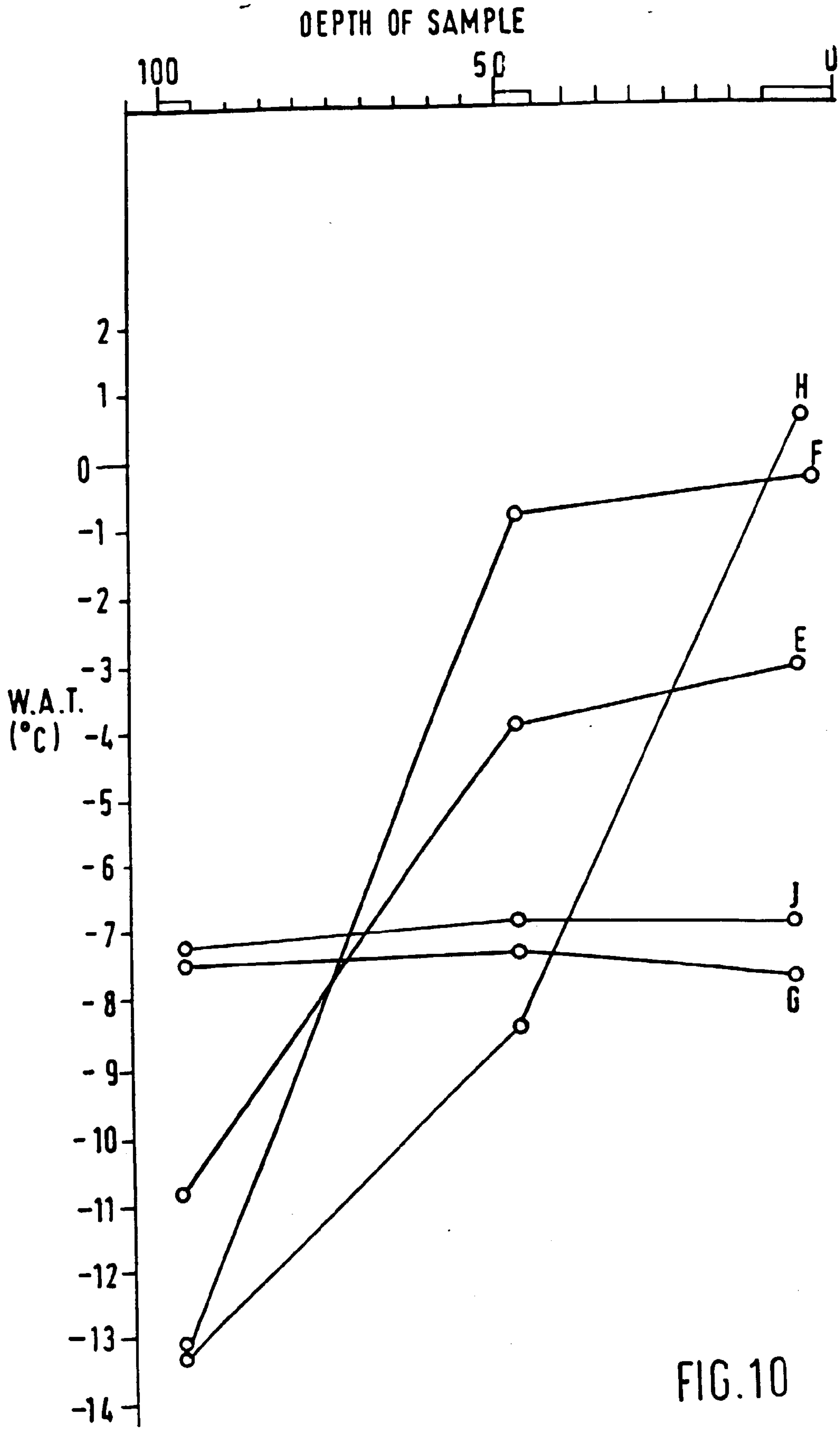
DEPTH OF SAMPLE
100
50
0
W.A.T.
(°C)
2
1
0
-1
-2
-3
-4
-5
-6
-7
-8
-9
-10
-11
-12
-13
-14
H
F
E
J
G

FIG.10